# EUROPEAN PATENT APPLICATION

(11) **EP 3 069 724 A1**
(43) Date of publication of application: **21.09.2016**
(21) Application number: 15159852.1
(22) Date of filing: 19.03.2015
(51) Int. Cl.: A61K 35/768, A61P 35/00

(54) **LUNG CANCER THERAPY WITH A RODENT PARVOVIRUS**

(71) Applicant: Deutsches Krebsforschungszentrum, 69120 Heidelberg (DE)
(72) Inventor: Marchini, Antonio, 69120 Heidelberg (DE); Bonifati, Serena, 87012 Castrovillari (IT); Rommelaere, Jean, 69118 Heidelberg (DE)
(74) Representative: Schüssler, Andrea

(57) **Abstract**

Described is the use of parvovirus for the treatment of lung cancer. The parvovirus is based on parvovirus H1 (H-1PV) or a related rodent parvovirus selected from the group consisting of LuIII, Mouse minute virus (MMV), Mouse parvovirus (MPV), Rat minute virus (RMV), Rat parvovirus or Rat virus (RV).

## Description

The present invention relates to the use of parvovirus H1 or a related rodent parvovirus for the treatment of lung cancer.

Lung cancer, also known as carcinoma of the lung or pulmonary carcinoma, is a malignant lung tumor characterized by uncontrolled cell growth in tissues of the lung. If left untreated, this growth can spread beyond the lung by process of metastasis into nearby tissue or other parts of the body. Most cancers that start in the lung, known as primary lung cancers, are carcinomas that derive from epithelial cells. The main primary types are small-cell lung carcinoma (SCLC) and non-small-cell lung carcinoma (NSCLC). The most common symptoms are coughing (including coughing up blood), weightloss, shortness of breath, and chest pains. Lung cancer may be seen on chest radiographs and computed tomography (CT) scans. The diagnosis is confirmed by biopsy which is usually performed by bronchoscopy or CT-guidance.

Treatment and long-term outcomes depend on the type of cancer, the stage (degree of spread), and the person's overall health, measured by performance status. Common treatments include surgery, chemotherapy, and radiotherapy. NSCLC is sometimes treated with surgery, whereas SCLC usually responds better to chemotherapy and radiotherapy. Overall, only 16.8% of people in the United States diagnosed with lung cancer survive five years after the diagnosis, while outcomes on average are worse in the developing world. Worldwide, lung cancer is the most common cause of cancer-related death in men and women, and was responsible for 1.56 million deaths annually, as of 2012.

The three main subtypes of NSCLC are adenocarcinoma, squamous-cell carcinoma and large-cell carcinoma. Nearly 40% of lung cancers are adenocarcinoma, which usually originates in peripheral lung tissue. Although most cases of adenocarcinoma are associated with smoking, adenocarcinoma is also the most common form of lung cancer among people who have smoked fewer than 100 cigarettes in their lifetimes ("never-smokers"). A subtype of adenocarcinoma, the bronchioloalveolar carcinoma, is more common in female never-smokers, and may have a better long term survival.

Squamous-cell carcinoma accounts for about 30% of lung cancers. They typically occur close to large airways. A hollow cavity and associated cell death are commonly found at the centre of the tumor. About 9% of lung cancers are large-cell carcinoma. These are so named because the cancer cells are large, with excess cytoplasm, large nuclei and conspicuous nucleoli.

In small-cell lung carcinoma (SCLC), the cells contain dense neurosecretory granules (vesicles containing neuroendocrine hormones), which give this tumor an endocrine/paraneoplastic syndrome association. Most cases arise in the larger airways (primary and secondary bronchi). These cancers grow quickly and spread early in the course of the disease. Sixty to seventy percent have metastatic disease at presentation. This type of lung cancer is strongly associated with smoking.

Due to the dissatisfying results achieved up to now as regards treatment of lung cancer therapy needs to be improved, e.g., as regards the extension of life span after diagnosis.

Therefore, it is the object of the present invention to provide means for an improved therapy of lung cancer.

According to the invention this is achieved by the subject matters defined in the claims.

The present invention is based on the applicant's findings that a number of lung cancer derived cell lines show higher susceptibility to parvovirus oncotoxicity in comparison with cell lines derived by other tumour entities (e.g. colon cancer). Thus, parvoviruses have tremendous therapeutic potential to treat lung cancers.

Viruses or armed vector derivatives that specifically kill neoplastically transformed cells (oncolysis) represent a promising approach for the treatment of dreadful cancer types for which conventional therapy is ineffective (e.g. pancreatic carcinomas, gliomas, hepatocellular carcinomas)(Russell et al, 2012). Some autonomous parvoviruses belong to the category of so called oncolytic viruses (Rommelaere et al, 2010). Parvoviruses are small (25-30 nm) non-enveloped particles containing a 5.1kb single-stranded DNA genome from which two nonstructural (NS1, NS2) and two capsid (VP1, VP2) proteins are expressed (Cotmore & Tattersall, 2007). Parvovirus H-1PV is able to trigger multiple cell death processes including apoptosis, necrosis and a cathepsin b dependent cell death pathway(Nuesch et al, 2012). Several members of the parvovirus genus (H-1PV, MVM, LuIII), whose natural hosts are rodents, are presently under consideration for cancer gene therapy applications due to their failure to transform host cells, capacity for asymptomatic infection of humans, and ability to preferentially propagate in (oncotropism) and kill (oncolysis) neoplastically transformed cells (Marchini et al, 2015). MVMp and H-1PV viruses have been shown to exert oncosuppressive activities in vivo, i.e. they are able to inhibit the formation of spontaneous, chemically or virally induced tumors in laboratory animals (reviewed in Marchini et al. 2015). Vectors based on a parvoviral expression cassette have been also used as anticancer agents. In addition, it is known that parvoviridae vectors may be employed as a vehicle in order to introduce a tumor suppressing nucleic acid, i.e. a tumor suppressor gene, into a patient suffering from cancer (US 2001/04420 A1).

In summary, H-1PV oncolytic potential has been demonstrated in in vitro and *in vivo* studies against a variety of tumors, including brain, breast, gastric, pancreatic, colon, melanoma and cervical cancers (recently reviewed in Marchini et al. 2015). However, the possibility to use H-1PV as anticancer agent against other tumor types has not yet been demonstrated. Therefore, in the experiments resulting in the present invention the NCI-60 cancer cell lines panel was screened for H-1PV sensitivity in order to acquire first *in vitro* evidence about the susceptibility of lung, ovarian, renal and/or prostate cancers to H-1PV oncotoxicity.

### Brief description of the drawings

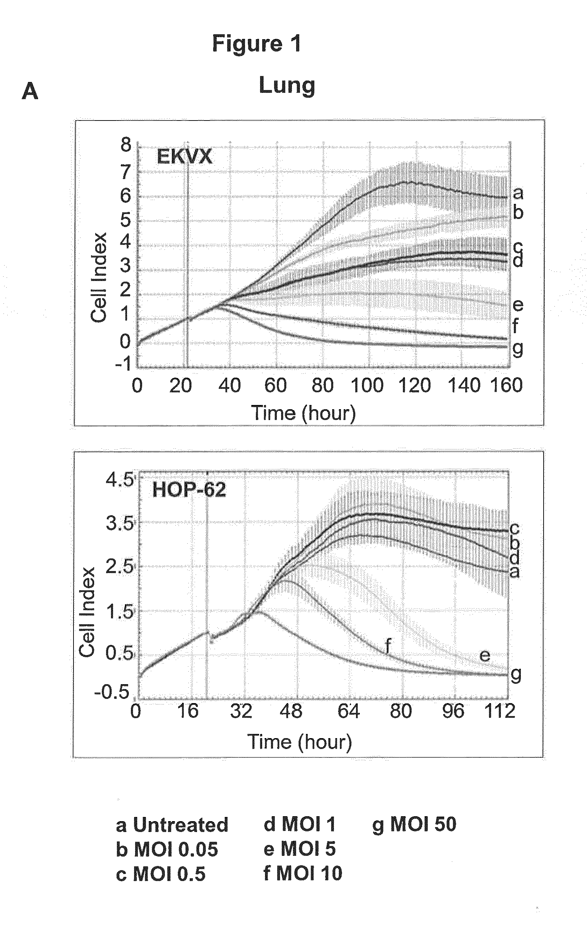

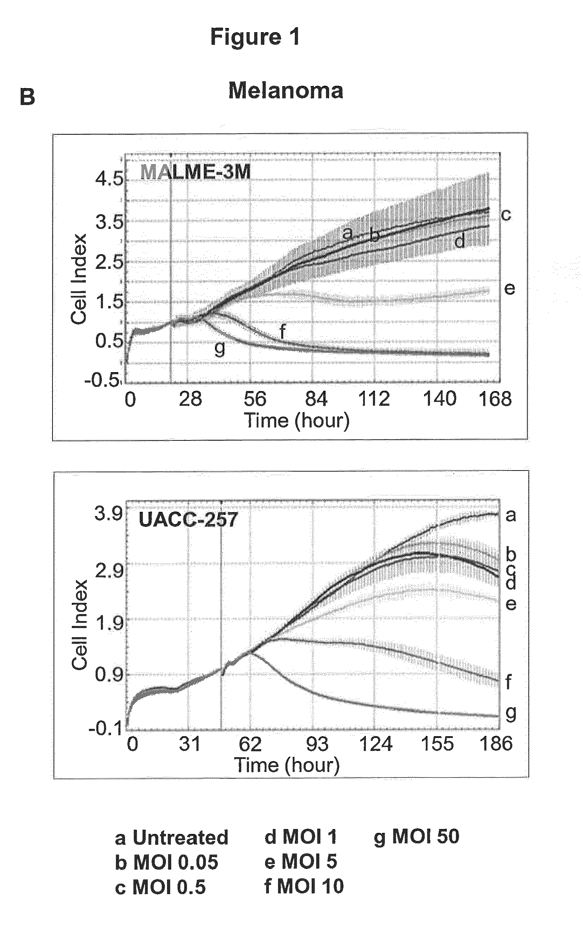

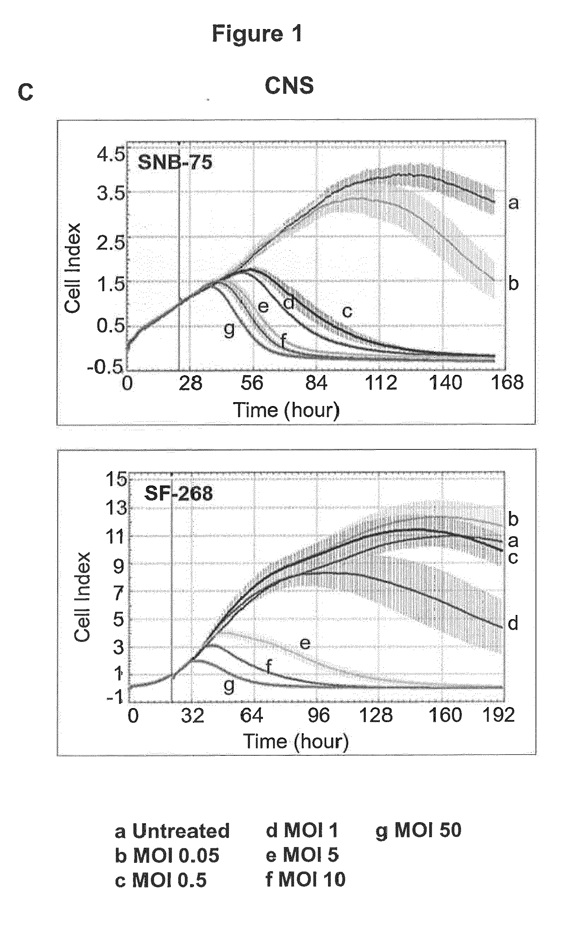

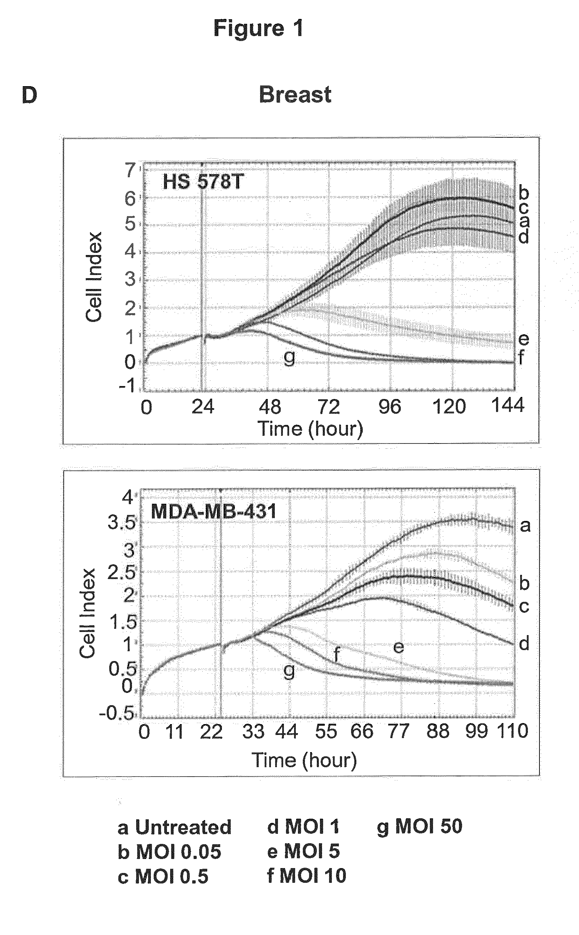

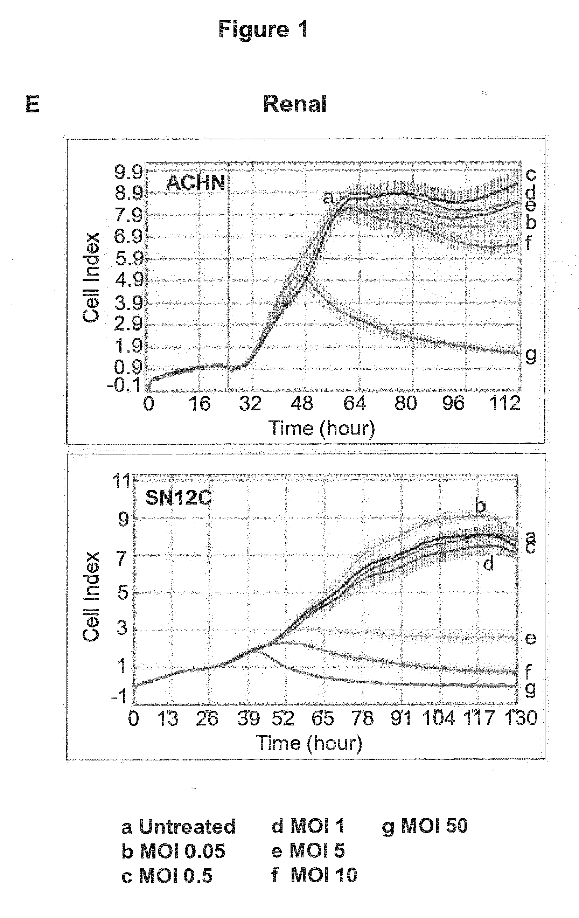

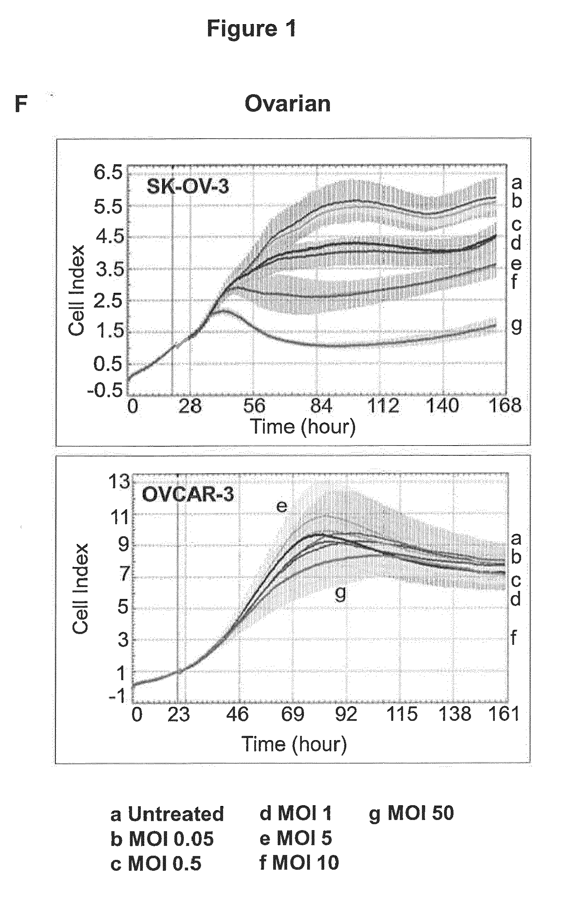

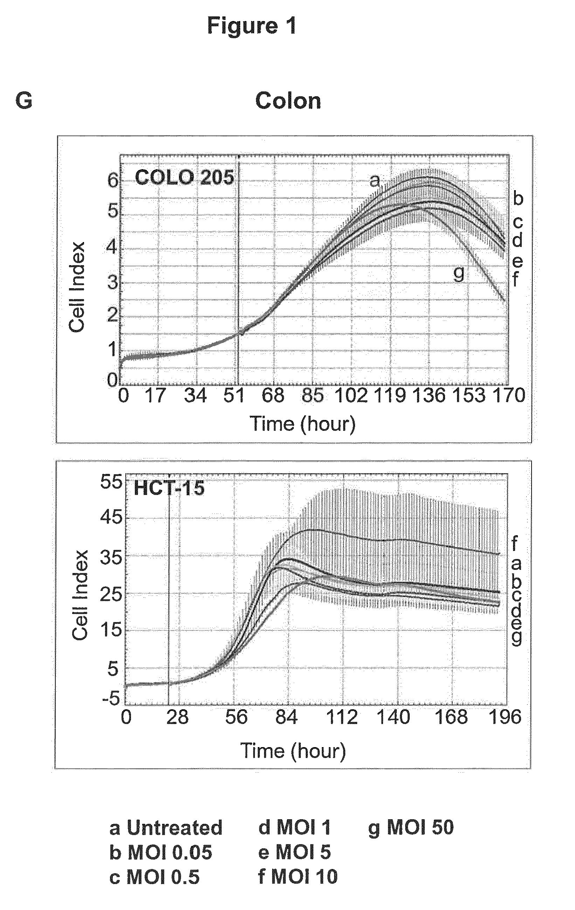

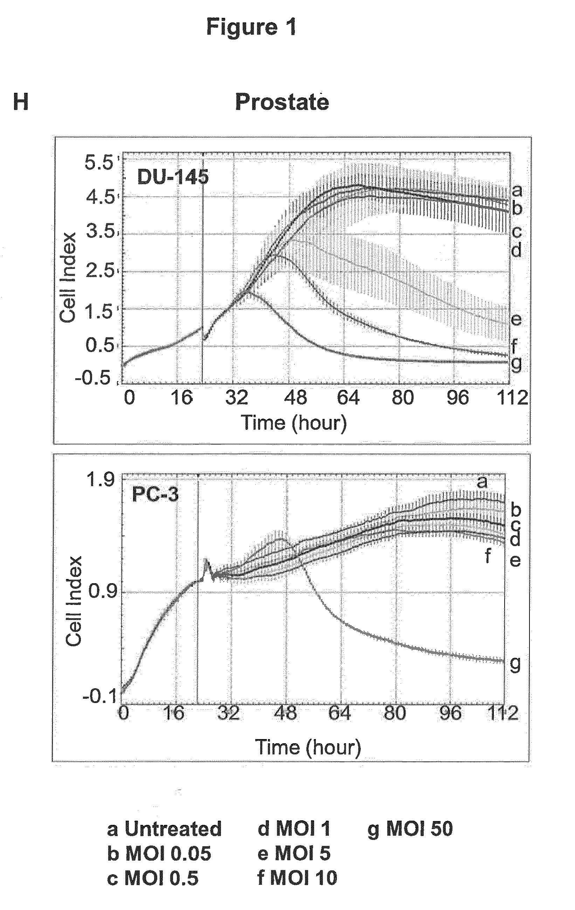

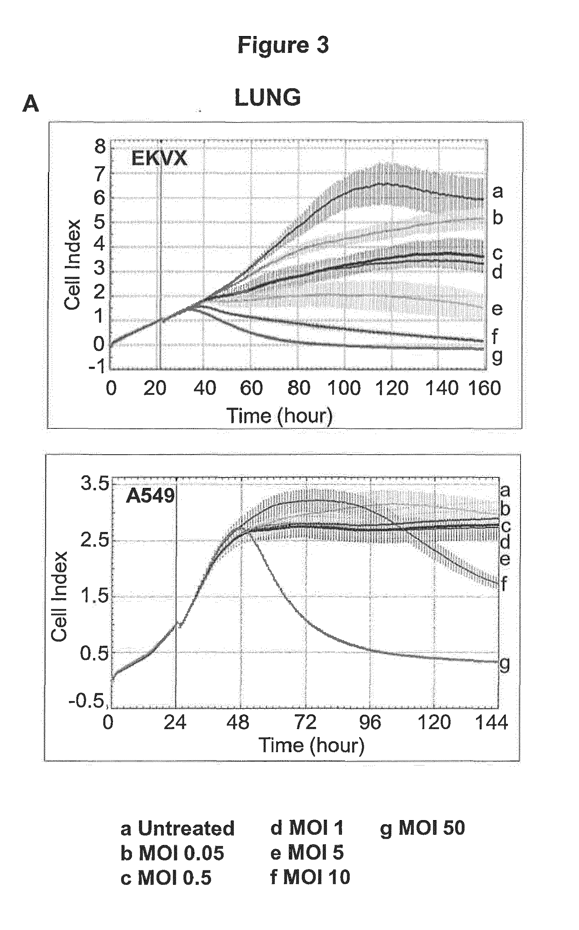

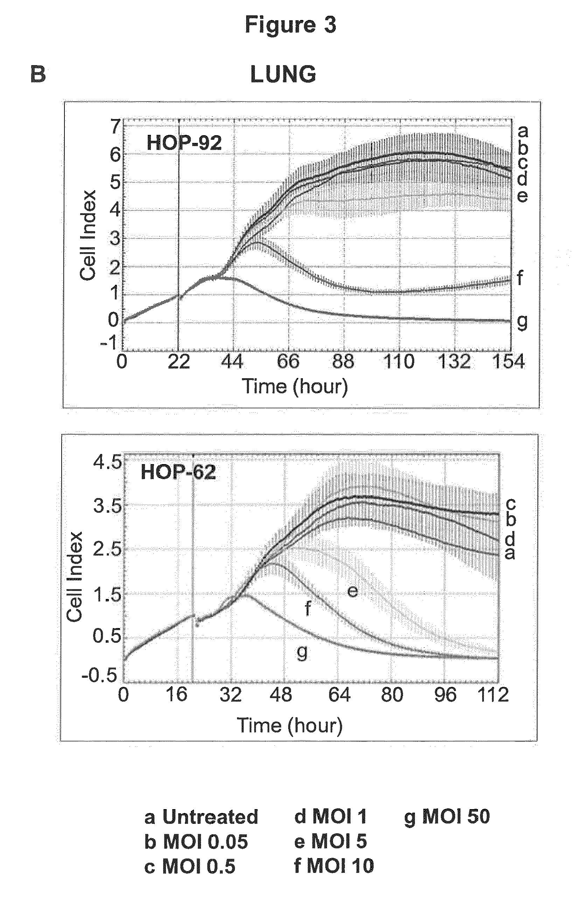

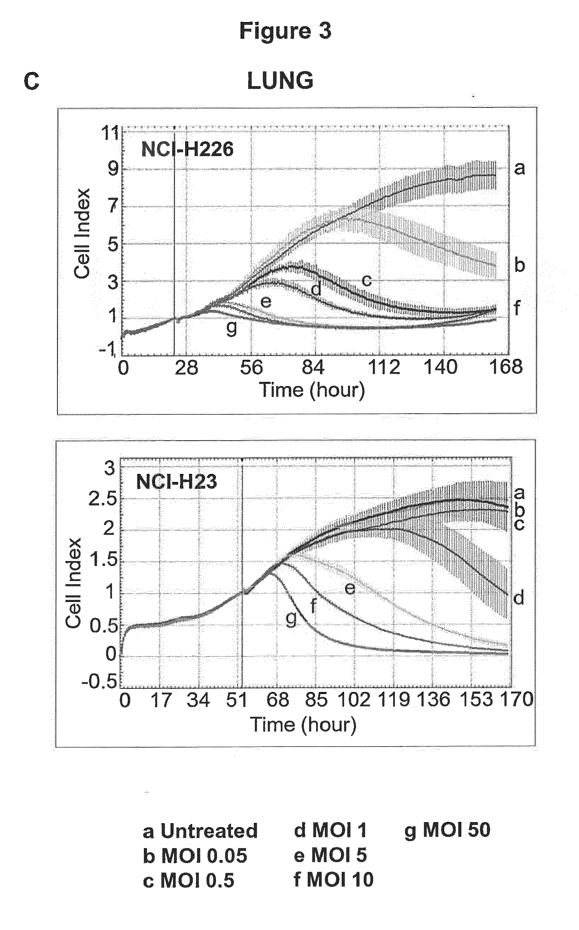

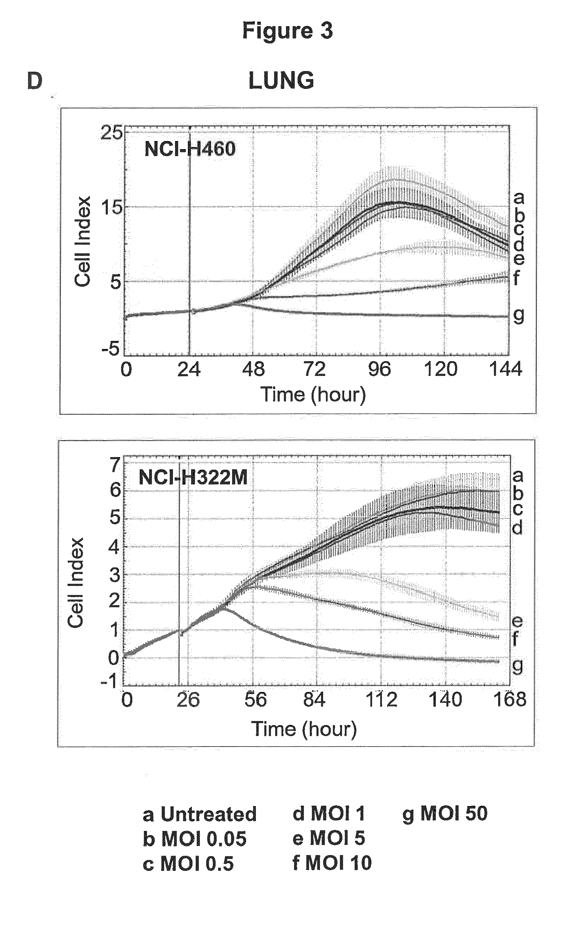

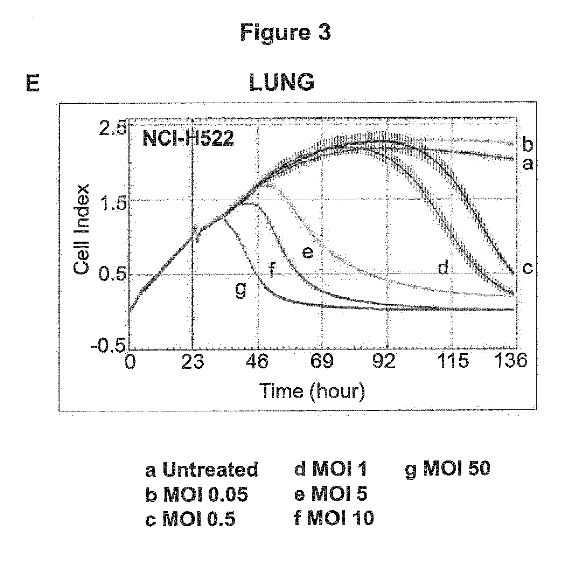

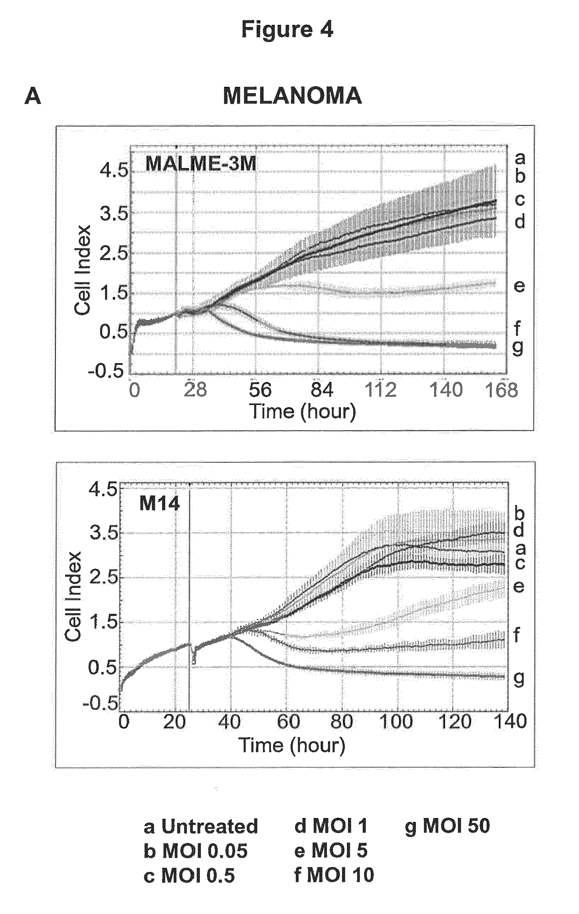

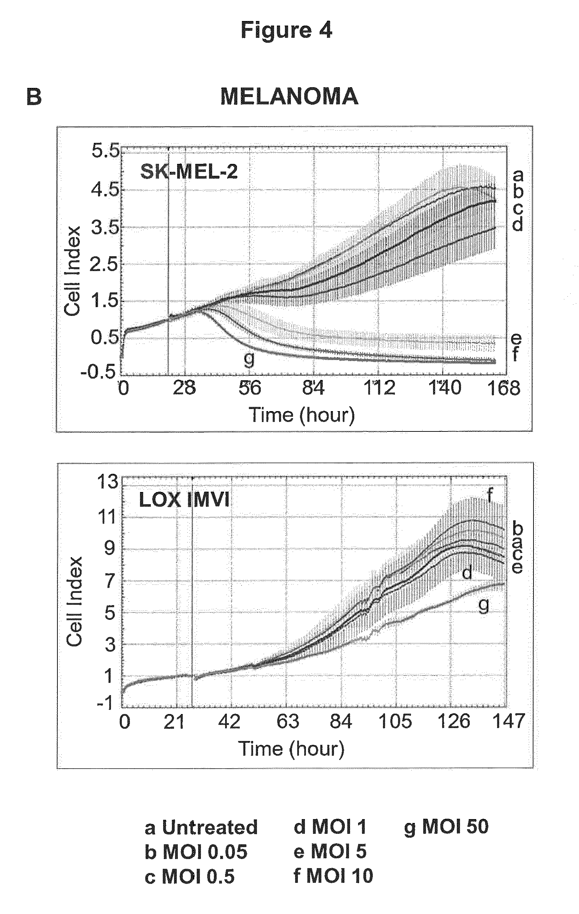

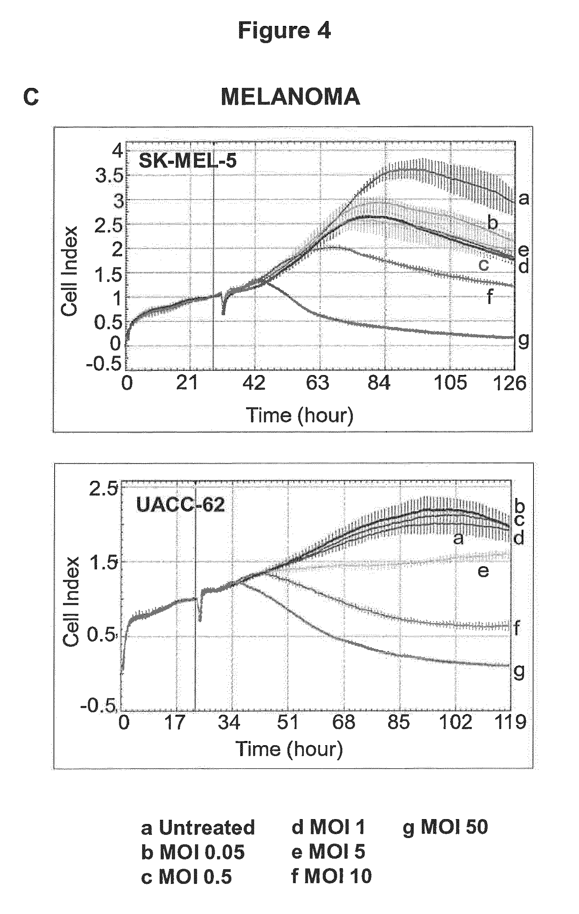

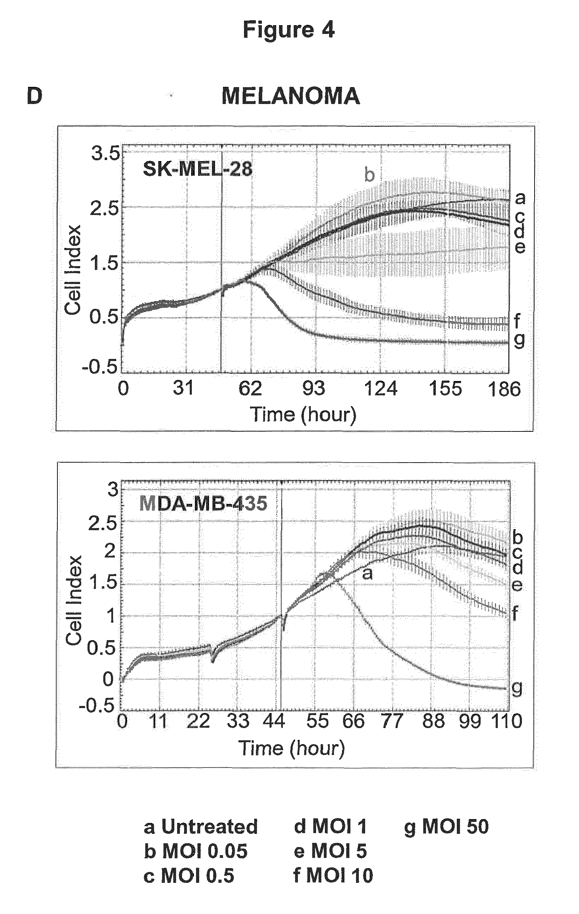

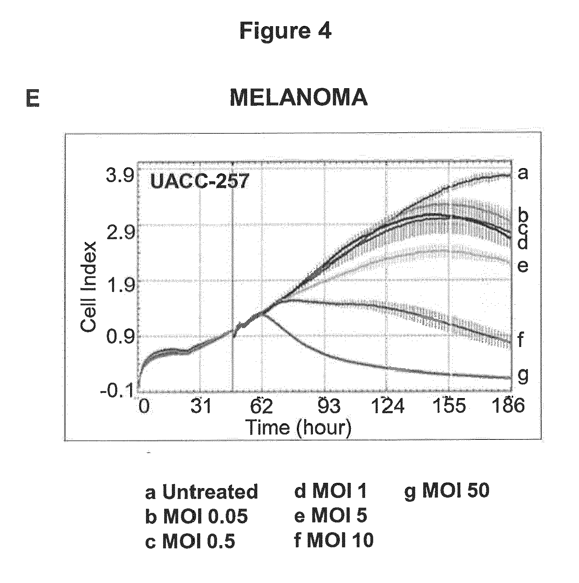

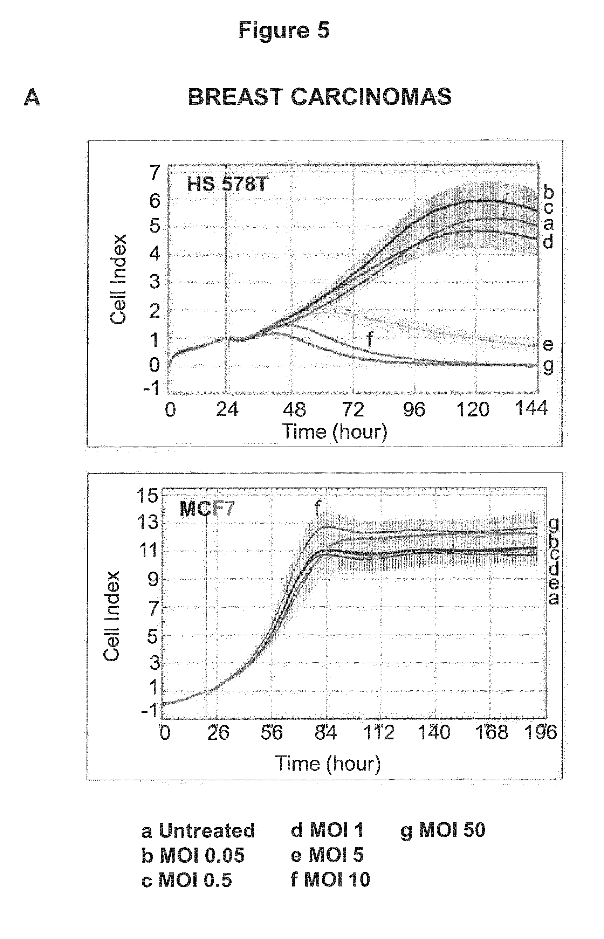

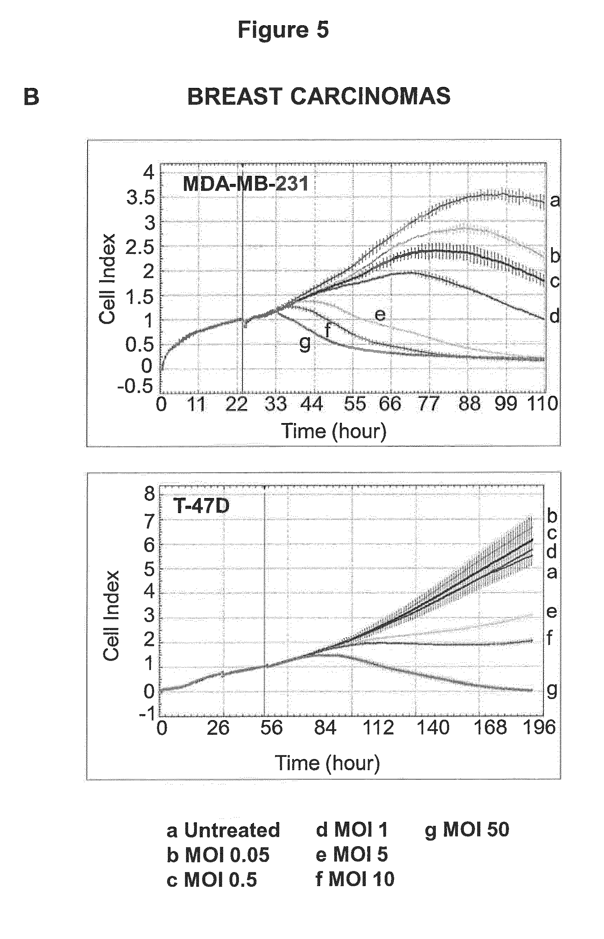

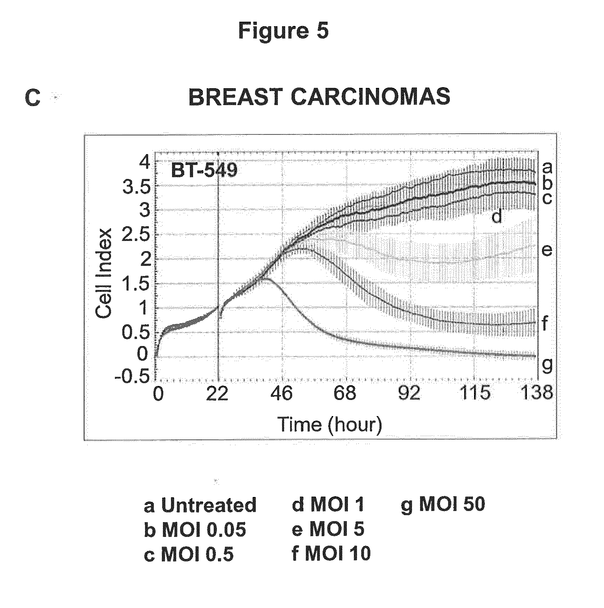

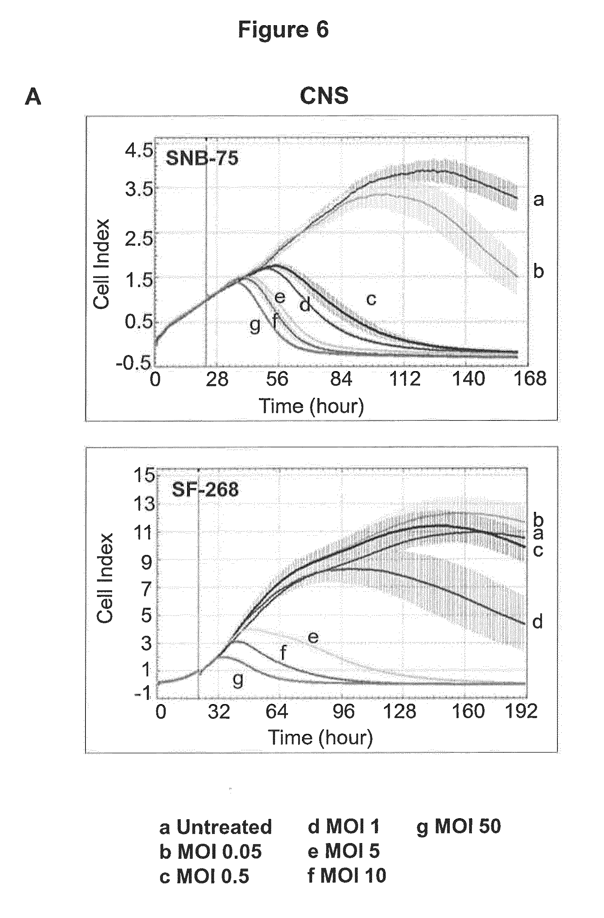

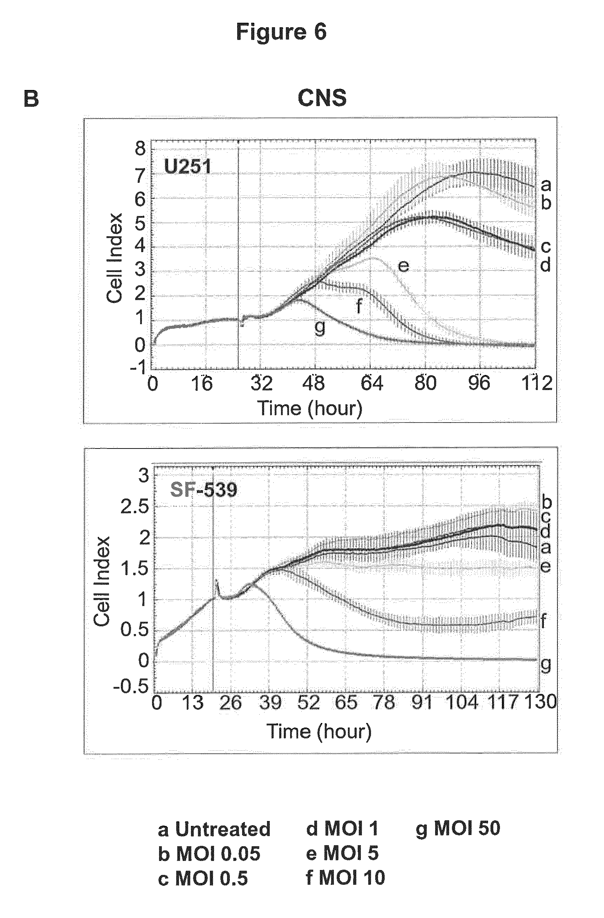

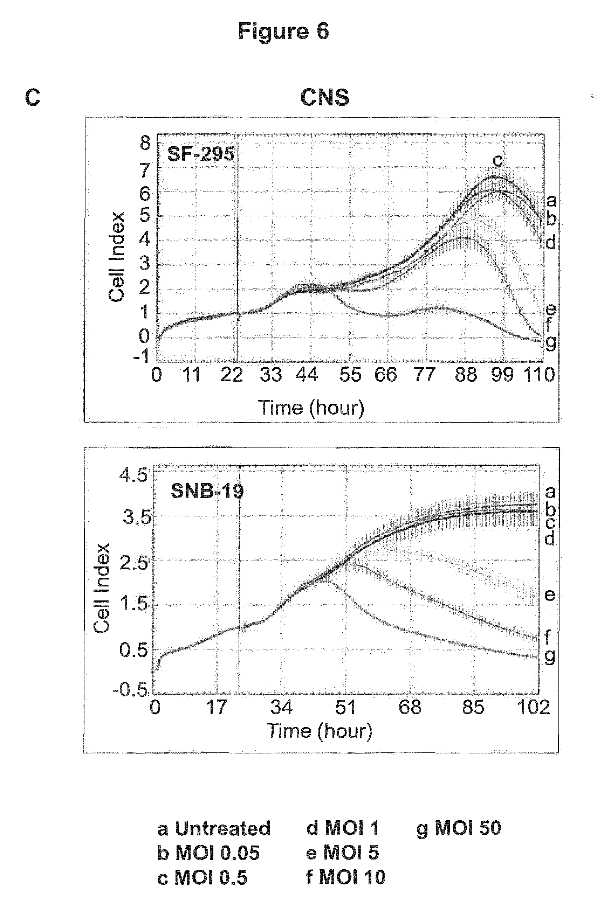

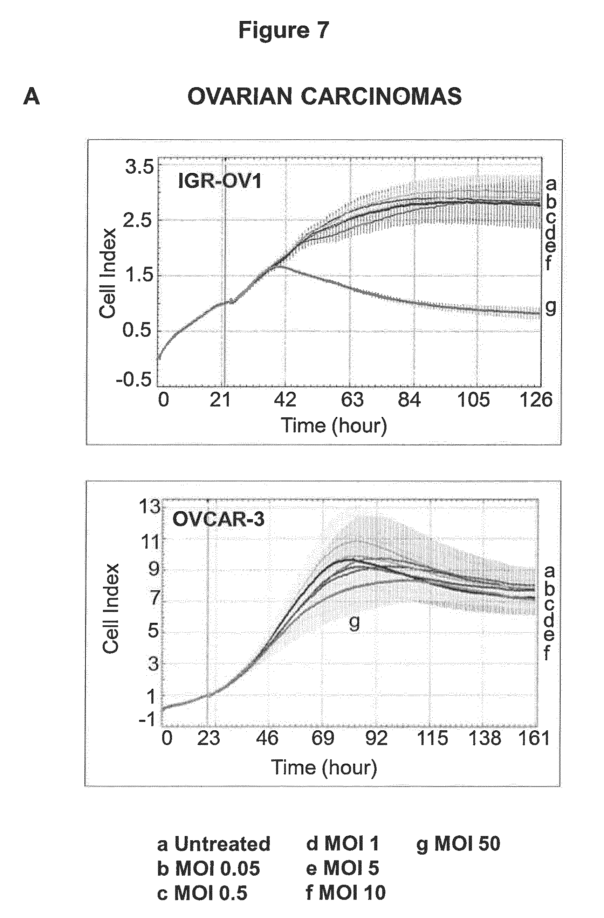

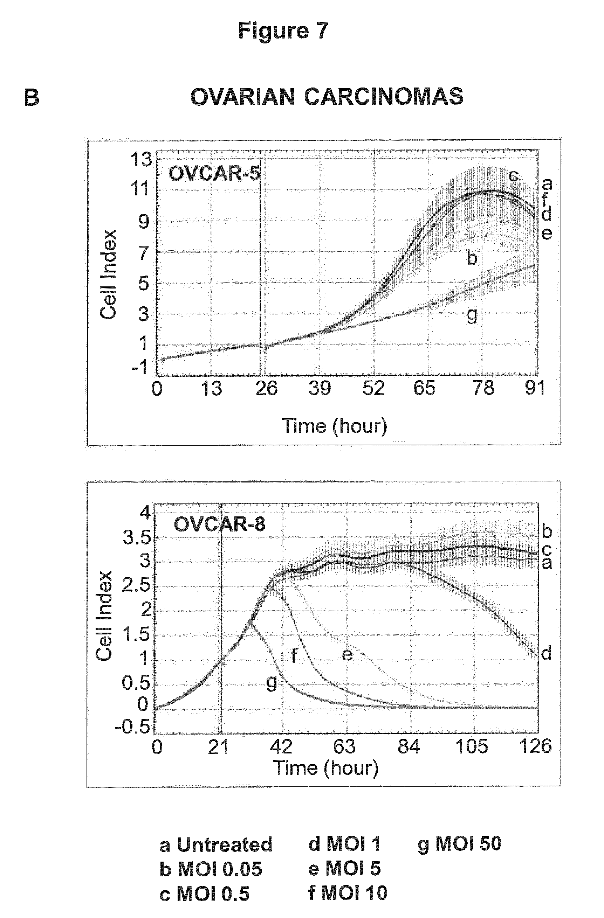

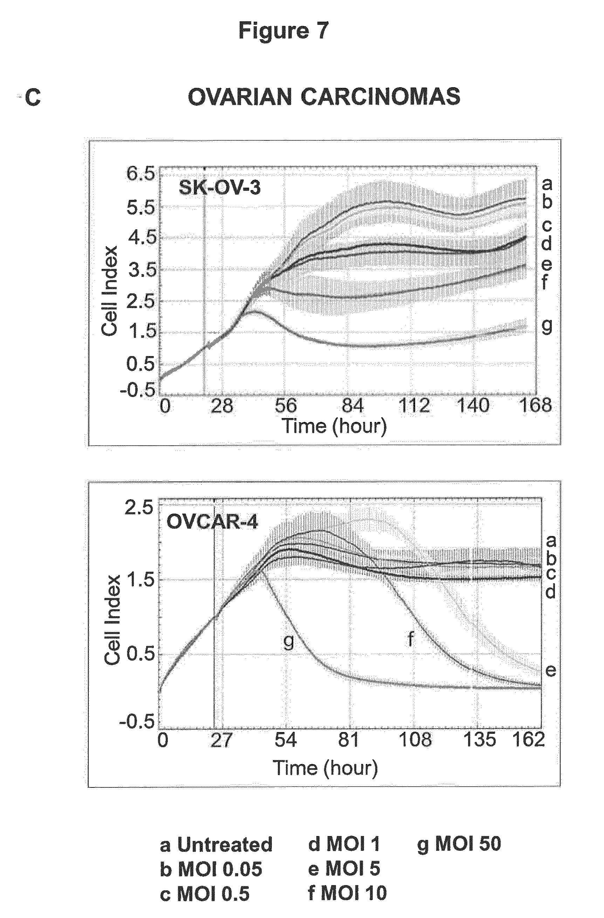

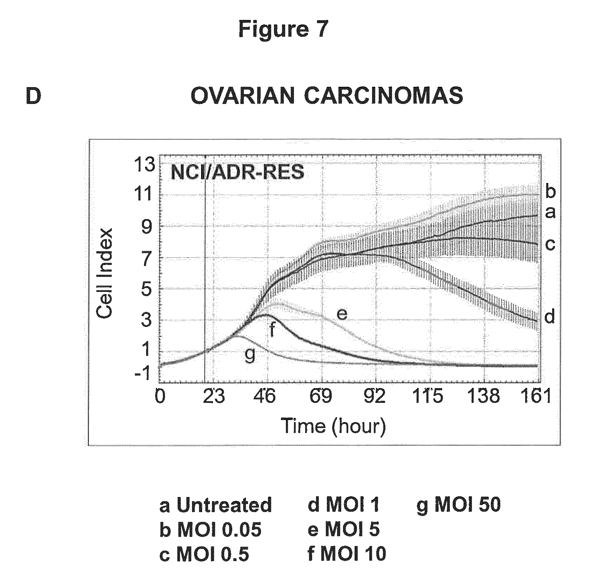

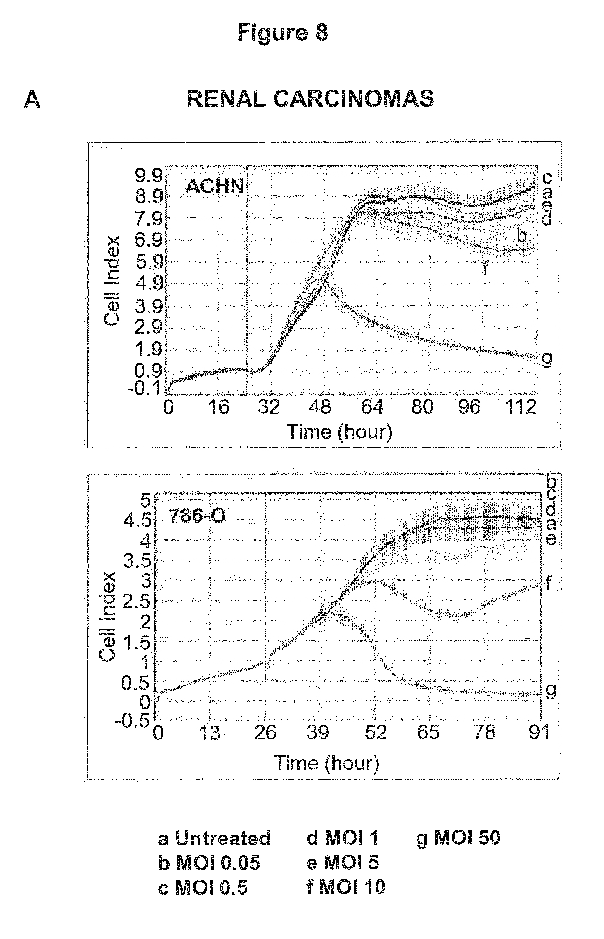

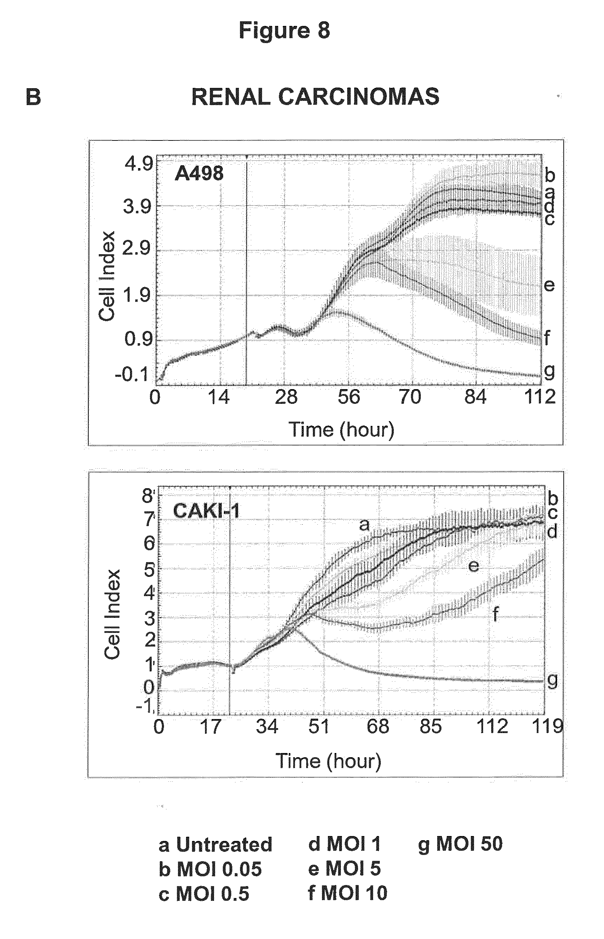

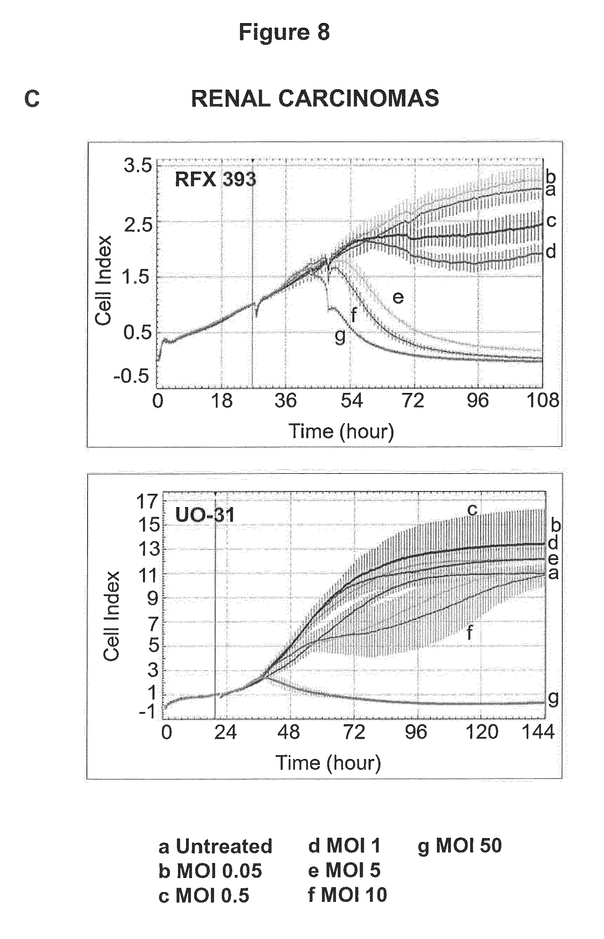

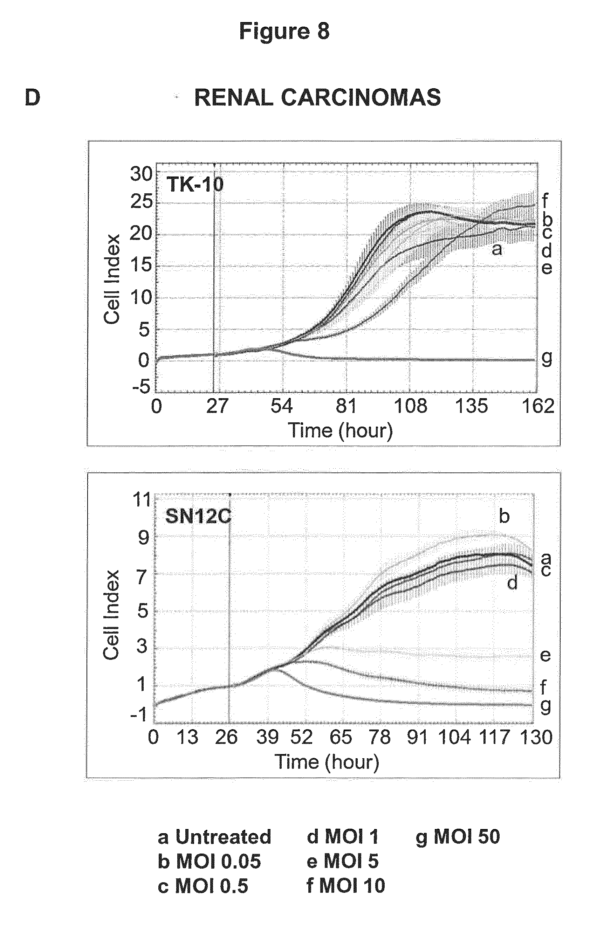

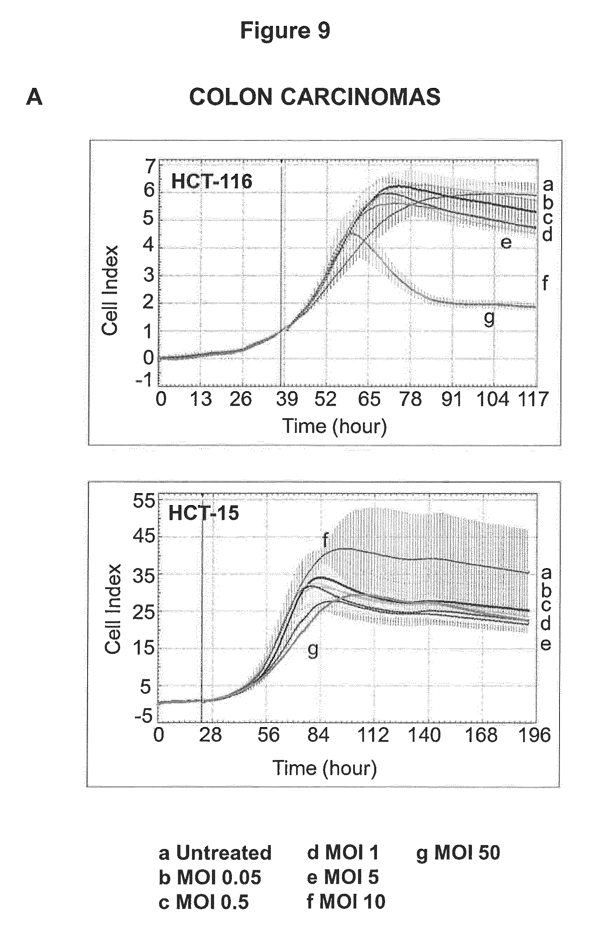

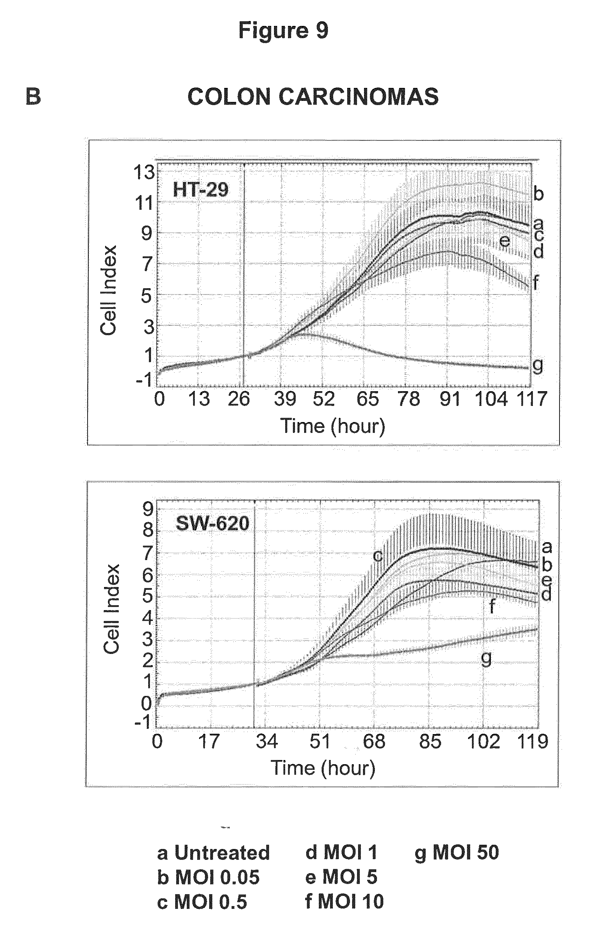

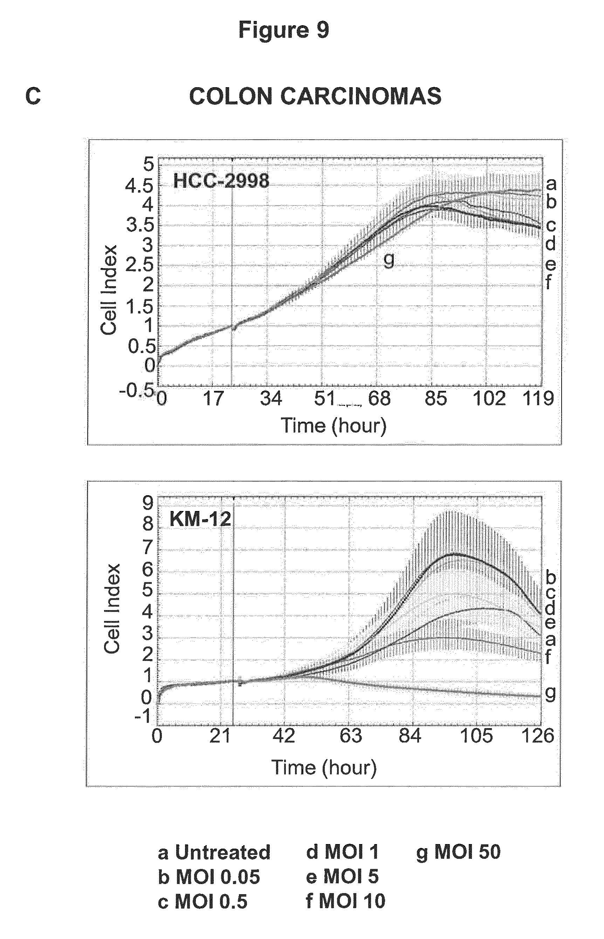

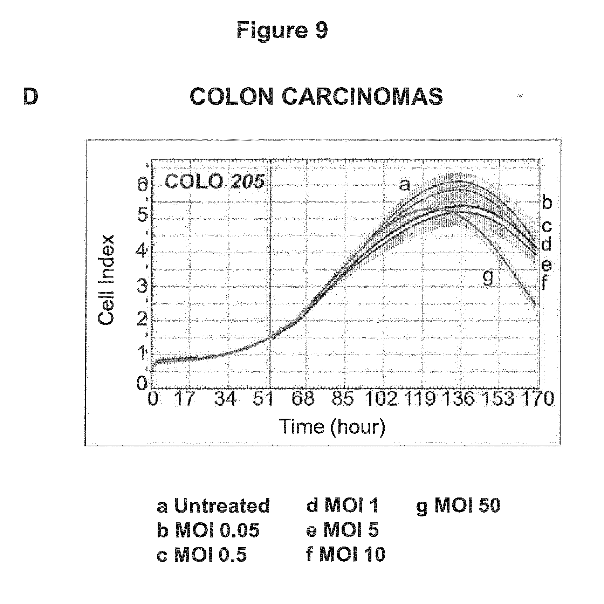

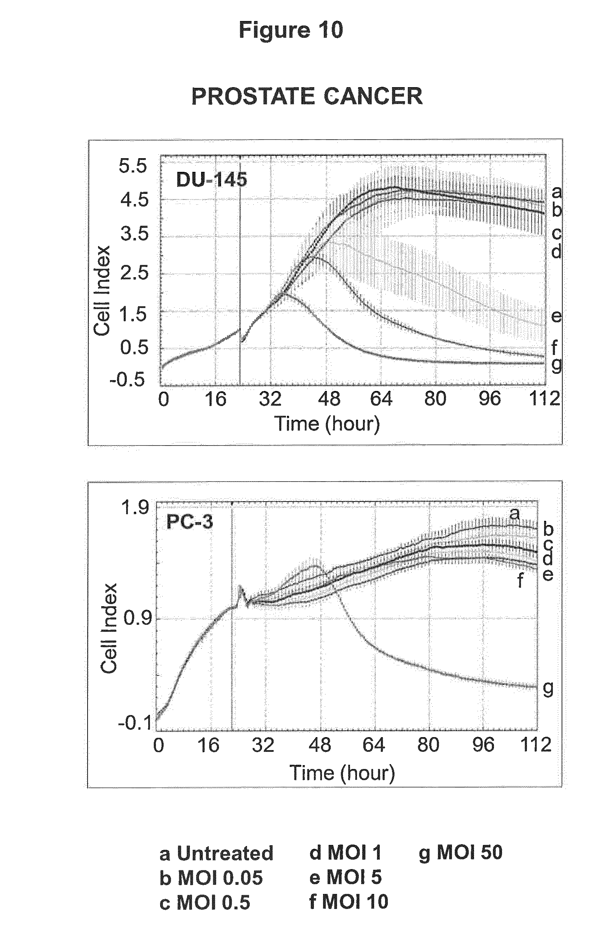

Figure 1. H-1PV oncolysis in the NCI-60 cell lines panel
   Cancer cells were seeded in a 96-well E-plate and either left untreated or infected with different MOIs (pfu/cell) of H-1PV. Cell growth and proliferation were monitored in real-time with the xCELLigence system, which every 30 minutes measures the amount of attached cells per well. These values are expressed as normalized cell index (CI) and plotted against time as a representation of cell growth curves. Each experimental condition was tested at least in triplicates and average values with standard deviation bars are represented. The vertical grey line indicates the time point of infection. Two cell lines, whose name is indicated in the upper left part of the graph, per tumor type are shown as examples. The complete results of the NCI-60 panel screening are reported in Figures 3 - 10.
   In Fig. 1 A - H the letters a - f at the right side mean
   a: Untreated
   b: MOI 0.05
   c: MOI 0.5
   d: MOI 1
   e: MOI 5
   f: MOI 10
   g: MOI 50
Figure 2. LDH and MTT assays in lung cancer and melanoma-derived cell lines belonging to the NCI-60 anel
   Cells were seeded in 96-well plates and, the next day, infected with increasing viral MOIs. At 48 hours post-infection, cells were processed for LDH (to measure cell lysis) and MTT (to determine cell viability). Average values were calculated from replicates and results from a representative experiment with standard deviation bars are shown.
Figure 3: Growth curves of lung cancer-derived cell lines untreated or infected with increasing viral MOIs
   All cell lines showed high sensitivity to H-1PV infection, being efficiently killed by the virus at low MOIs (≤10). The A549 cell line represents the only exception, as a MOI of50 pfu/cell was needed to induce cytotoxic effects at 48-72 hours post-infection.
   In Fig. 3 A - E the letters a - f at the right side mean
   a: Untreated
   b: MOI 0.05
   c: MOI 0.5
   d: MOI 1
   e: MOI 5
   f: MOI 10
   g: MOI 50
Figure 4: Growth curves of melanoma-derived cell lines untreated or infected with increasing viral MOIs
   Cytostatic effects were observed in most of the cell lines upon H-1PV infection: in M14, MALME-3M, UACC-62, SK-MEL-28 and UACC-257 cell lines, reduced cell growth was observed when the virus was used at MOI of 5 pfu/cell, while at higher MOIs H-1PV induced cell killing. In SK-MEL-2 and SK-MEL-5 cell growth retardation was detected at lower MOIs, varying from 0.5 to 1 pfu/cell, and cytotoxicity was observed in cells infected with MOI 5 or 10. Only LOX IMVI cells showed resistance to H-1PV cytotoxicity.
   In Fig. 4 A - E the letters a - f at the right side mean
   a: Untreated
   b: MOI 0.05
   c: MOI 0.5
   d: MOI 1
   e: MOI 5
   f: MOI 10
   g: MOI 50
Figure 5: Growth curves of breast cancer cell lines untreated or infected with increasing viral MOIs
   Breast cancer can be considered as a potential target of H-1PV oncolysis since three out of five cell lines showed sensitivity at the viral MOI of 5 pfu/cell and in one cell line (MDA-MB-231) infection with low MOIs (0.05-0.5 pfu/cell) was associated with cytostatic effects. Only one cell line, MCF7, was resistant to H-1PV-induced cytotoxicity.
   In Fig. 5 A - C the letters a - f at the right side mean
   a: Untreated
   b: MOI 0.05
   c: MOI 0.5
   d: MOI 1
   e: MOI 5
   f: MOI 10
   g: MOI 50
Figure 6: Growth curves of central nervous system (CNS)-derived cell lines untreated or infected with increasin viral MOIs
   Three cell lines (SF-539, SF-295, SNB-19) were sensitive at the viral MOI of 5 pfu/cell, while the other three were efficiently killed by lower viral amounts. These results confirm previous published data showing H-1PV oncolytic potential against this tumor type.
   In Fig. 6 A - C the letters a - f at the right side mean
   a: Untreated
   b: MOI 0.05
   c: MOI 0.5
   d: MOI 1
   e: MOI 5
   f: MOI 10
   g: MOI 50
Figure 7: Growth curves of ovarian cancer cell lines untreated or infected with increasing viral MOIs
   The screening revealed that OVCAR-3 and OVCAR-5 were resistant to H-1PV cytotoxicity (a slight cytostatic effect was detected in OVCAR-5), IGR-OV1 were efficiently killed when the virus was used at MOI of 50 pfu/cell, while in the remaining four cell lines MOIs of 5 pfu/cell or lower were sufficient to induce cytotoxic effects.
   In Fig. 7 A - D the letters a - f at the right side mean
   a: Untreated
   b: MOI 0.05
   c: MOI 0.5
   d: MOI 1
   e: MOI 5
   f: MOI 10
   g: MOI 50
Figure 8: Growth curves of renal carcinomas-derived cell lines untreated or infected with increasing viral MOIs
   When used at MOI ≥ 5 pfu/cell, H-1PV was able to kill four cell lines (786-0, A498, RFX 393 and SN12C), while the highest MOI used in the experiment was needed to efficiently kill ACHN, CAKI-1, UO-31 AND TK-10 cells. Based on these results, the inventors concluded that tumors of renal origin are medium sensitive to H-1PV oncolysis.
   In Fig. 8 A - D the letters a - f at the right side mean
   a: Untreated
   b: MOI 0.05
   c: MOI 0.5
   d: MOI 1
   e: MOI 5
   f: MOI 10
   g: MOI 50
Figure 9: Growth curves of colon cancer-derived cell lines untreated or infected with increasing viral MOIs
   The screening revealed the low permissivity of colon cancer cell lines to H-1PV infection, as cytotoxicity was observed at the highest MOI used (50 pfu/cell) in four cell lines (with cytostatic effects observed at MOI 10 in SW-620 and HT-29 cells), while in other three cell lines cell growth was not affected by virus infection in the experimental conditions tested.
   In Fig. 9 A - D the letters a - f at the right side mean
   a: Untreated
   b: MOI 0.05
   c: MOI 0.5
   d: MOI 1
   e: MOI 5
   f: MOI 10
   g: MOI 50
Figure 10: Growth curves of prostate cancer cell lines untreated or infected with increasing viral MOIs
   DU-145 cells showed sensitivity to H-1PV infection when the virus was used at MOI 5, while PC-3 cells seemed to be more resistant to the virus-induced effects. Since in the NCI-60 panel prostate cancer is represented by only two cell lines, it was not possible to make any solid conclusion about the H-1PV oncolytic potential in this tumor type.

The present invention provides parvovirus H1 (H-1PV) or a related rodent parvovirus for use in a method of treating lung cancer.

The term "parvovirus" (or "parvotherapeutic agent") as used herein comprises wild-type or modified replication-competent derivatives thereof. Suitable parvoviruses which can be used for actively producing said parvoviruses and which are useful for therapy, are readily determinable within the skill of the art based on the disclosure herein, without undue empirical effort.

According to the present invention, the parvovirus of the composition includes parvovirus H1 (H-1PV) or a related parvovirus such as LuIII, Mouse minute virus (MMV), Mouse parvovirus (MPV), Rat minute virus (RMV), Rat parvovirus (RPV) or Rat virus (RV).

According to the invention the parvovirus (or parvotherapeutic agent) is present in an effective dose and combined with a pharmaceutically acceptable carrier. "Pharmaceutically acceptable" is meant to encompass any carrier, which does not interfere with the effectiveness of the biological activity of the active ingredients and that is not toxic to the patient to whom it is administered. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and can be administered to the subject at an effective dose.

An "effective dose" refers to amounts of the active ingredients that are sufficient to affect the course and the severity of the disease, leading to the reduction or remission of such pathology. An "effective dose" useful for treating and/or preventing these diseases or disorders may be determined using methods known to one skilled in the art.

Additional pharmaceutically compatible carriers can include gels, bioadsorbable matrix materials, implantation elements containing the therapeutic agent, or any other suitable vehicle, delivery or dispensing means or material(s).

In a preferred embodiment the parvovirus is in combination with a chemotherapeutic agent.

Administration may be effected by different ways, e.g. by intravenous, intraperitoneal, subcutaneous, intramuscular, topical or intradermal administration. The route of administration, of course, depends on the kind of therapy and the kind of compounds contained in the pharmaceutical composition. A preferred route of administration is intravenous administration. The dosage regimen of the parvotherapeutic agent (and the chemotherapeutic agent) is readily determinable within the skill of the art, by the attending physician based on patient data, observations and other clinical factors, including for example the patient's size, body surface area, age, sex, the particular parvovirus, cell, chemotherapeutic agent etc. to be administered, the time and route of administration, the lung cancer type and characteristics, general health of the patient, and other drug therapies to which the patient is being subjected.

If the parvotherapeutic agent(s) according to the invention comprises infectious virus particles with the ability to penetrate through the blood-brain barrier, treatment can be performed or at least initiated by intravenous injection of the parvotherapeutic agent, e.g., H1 virus. A preferred route of administration is intratumoral administration.

Since long-term intravenous treatment is susceptible to becoming inefficient as a result of the formation of neutralizing antibodies to the parvotherapeutic agent, different modes of administration can be adopted after an initial regimen intravenous viral administration, or such different administration techniques, e.g., intratumoral virus administration, can be alternatively used throughout the entire course of parvoviral treatment.

As another specific administration technique, the parvotherapeutic agent (virus, vector and/or- cell agent) can be administered to the patient from a source implanted in the patient. For example, a catheter, e.g., of silicone or other biocompatible material, can be connected to a small subcutaneous reservoir (Rickham reservoir) installed in the patient during tumor removal or by a separate procedure, to permit the parvotherapeutic composition to be injected locally at various times without further surgical intervention.

Administration of the parvotherapeutic agents or compositions can also be performed by continuous infusion of viral particles or fluids containing viral particles through implanted catheters at low flow rates using suitable pump systems, e.g., peristaltic infusion pumps or convection enhanced delivery (CED) pumps.

As yet another method of administration of the parvotherapeutic composition is from an implanted article constructed and arranged to dispense the parvotherapeutic agent to the desired cancer tissue. For example, wafers can be employed that have been impregnated with the parvotherapeutic composition, e.g., parvovirus H1, wherein the wafer is attached to the edges of the resection cavity at the conclusion of surgical tumor removal. Multiple wafers can be employed in such therapeutic intervention. Cells that actively produce the parvotherapeutic agent, e.g., parvovirus H1, or H1 vectors, can be injected into the tumor, or into the tumoral cavity after tumor removal.

Patients treatable by the parvovirus according to the invention include humans as well as non-human animals. Examples of the latter include, without limitation, animals such as cows, sheep, pigs, horses, dogs, and cats.

Chemotherapeutic agents useful in combination with the parvovirus for the purposes of the present invention include all chemical compounds that are effective in inhibiting tumor growth. The administration of chemotherapeutic agents can be accomplished in a variety of ways (see above) including systemically by the parenteral and enteral routes. Preferably, the parvovirus and the chemotherapeutic agent are administered as separate compounds.

In a further preferred embodiment, the parvovirus is administered after the chemotherapeutic agent. The preferred period of time between administration of the chemotherapeutic agent and the parvovirus is from 14 to 35 days.

Examples of suitable chemotherapeutic agents include alkylating agents, for example, nitrogen mustards, ethyleneimine compounds and alkyl sulphonates; antimetabolites, for example, folic acid, purine or pyrimidine antagonists, mitotic inhibitors, for example, vinca alkaloids and derivatives of podophyllotoxin; cytotoxic antibiotics; compounds that damage or interfere with DNA expression; and growth factor receptor antagonists. Particular examples of chemotherapeutic agents suitable for the combined therapy include cisplatin, dacarbazine (DTIC), dactinomycin, mechlorethamine (nitrogen mustard), streptozocin, cyclophosphamide, carmustine (BCNU), lomustine (CCNU), doxorubicin (adriamycin), daunorubicin, procarbazine, mitomycin, cytarabine, etoposide, methotrexate, 5-fluorouracil, vinblastine, vincristine, bleomycin, paclitaxel (taxol), docetaxel (taxotere), aldesleukin, asparaginase, busulfan, carboplatin, cladribine, dacarbazine, floxuridine, fludarabine, hydroxyurea, ifosfamide, leuprolide, megestrol, melphalan, mercaptopurine, plicamycin, mitotane, pegaspargase, pentostatin, pipobroman, plicamycin, streptozocin, tamoxifen, teniposide, testolactone, thioguanine, thiotepa, uracil mustard, vinorelbine, chlorambucil and combinations thereof. Particularly preferred chemotherapeutic agents are Gemcitabine and Temozolodine.

The below examples explain the invention in more detail.

### Example 1

### Materials and Methods

### (A) Cell culture

Cell lines belonging to the NCI 60 cell line collection were cultivated as adherent monolayer cultures at 37°C temperature, 5% CO₂ atmosphere and 90% humidity in RPMI supplemented with 10% FCS and 2 mM L-glutamine.

### (B) Virus production and titration

Wild-type H-1PV was produced on human NBK324K cells by infecting seeded cells (1×10⁶ cells in 10-cm dish) in serum-and antibiotics-free medium with H-1PV at multiplicity of infection (MOI) of 0.003 pfu/cell. Cells were cultured for additional 4 hours in medium supplemented with 10% FCS at 37°C. Afterwards, cells were transferred to 15-cm dishes and cultured until cell lysis became apparent. Cells were then harvested in medium and lysed in 10 ml of VTE buffer (50 mM Tris-HCl, 0.5 mM EDTA, pH 8.7). Cell integrity was disrupted by three freeze and thaw cycles and, after overnight incubation at -20°C, a purification gradient was generated by overlapping 15%/25%/40%/60% iodixanol solutions to the virus suspension. Subsequently, vacuum centrifugation at 50,000 K for 2 hours at 4°C was performed, and the virus was extracted from the iodixanol 40% fraction using a syringe.

Virus titers were determined by plaque assays on NBK324K cells. Cells were seeded in 6-cm dishes at a density of 5x10⁵ cells per dish. The day after, cells were infected in MEM medium without serum or antibiotics containing serial dilutions of H-1PV stock for 1 hour at 37°C with gentle shaking every 10 min to ensure equal distribution of the virus. Afterwards, virus suspension was aspirated and cells were cultured for 5-6 days in 5 ml MEM supplemented with 5% FCS and 0.65% Bacto™Agar. Plaque formation was determined after addition of 3 ml of PBS 1x solution containing 0.18% Neutral Red and 0.8% Bacto™Agar. Next day, agar layers were removed, cells were fixed in 4% paraformaldehyde for 10 min, and numbers of plaques were counted from duplicates. H-1PV titers were thereby expressed as plaque forming units per ml.

### (C) Real-Time detection of cell proliferation/viability

Cells were seeded on a 96-well E-Plate (Roche Diagnostics Deutschland GmbH, Mannheim, Germany) at a density of 4,000-16,000 cells/well (according to cell doubling rate). 24 h to 72 h later, cells were infected with increasing amounts of H-1PV wild-type, ranging from multiplicity of infection (MOI = pfu/cell) of 0.05 to 50 pfu/cell (0, 0.05, 0.25, 0.5, 1, 5, 10 and 50). Growth of untreated and H-1PV infected cells was monitored in real-time for 5-7 days and expressed as normalized cell index (CI), a parameter proportional to the number of attached cells per well and, therefore, strictly correlated with cell proliferation rate using the xCELLigence System (Roche Diagnostics Deutschland GmbH, Mannheim, Germany). Cell proliferation was monitored in real time, every 30 min. The growth curves shown represent the average of at least three replicates with relative standard deviations. The 6 in suspension-growing leukaemia cell lines were excluded from the screening because they are not compatible with the xCELLigence system, which can only monitor the growth of adherent cell lines.

### (D) Lactate dehydrogenase (LDH) assay

To determine cell lysis upon virus infection, cells were seeded on a 96-well plate at a density of 1,500-6,000 cells/well in 50 µl of culture medium supplemented with 10% heat-inactivated FCS. The day after, 50 µl of medium, without serum or antibiotics, containing H-1PV at the desired concentration were added on top of the cells. At 72-96 hours post treatment, the release of the lactate dehydrogenase (LDH) was measured by using the CytoTox96^{®} Non-Radioactive Cytotoxicity Assay (Promega) according to manufacturer's instructions, using an enzyme-linked immunosorbent assay (ELISA) plate reader at 492 nm. For each experimental condition, seven replicates were prepared, out of which three were used for calculating the total cell lysis in the presence of detergent. The percentage of lysed cells upon virus treatment was calculated as a ratio of the LDH activity in the sample to the LDH activity after total lysis of the corresponding cultures.

### (E) MTT assay

The same cell cultures in which LDH activity was determined (see above) were used to assess cell viability by measuring the conversion of 3-(4,5-dimethylthiazol-2-yl)-2,5-diphenyl-2H-tetrazolium bromide (MTT) into an insoluble purple formazan product through a colorimetric reaction. This conversion can only be carried out by active mitochondrial dehydrogenases and, therefore, this parameter can be used as a measurement of cell proliferation/viability. 10 µl of 5 mg/ml MTT were added to 50 µl of culture samples. After incubation for 3 hours at 37°C, the supernatant was aspirated and plates were dried at 37°C overnight. To solubilize the formazan product, cells were then incubated with 100 µl of isopropanol for 20 min with moderate shaking and the absorbance was measured by using an ELISA plate reader at 595 nm. Viability of treated cells was expressed as a ratio of the measured absorbance (average of four replicates per condition) to the corresponding absorbance of untreated cells (defined as 100%).

### (F) Determination of the EC50 value relative to H-1PV infection in the NCI-60 cell lines screening

A two-step data analysis approach (Kinsner-Ovaskainen et al, 2013) was applied to derive, for each NCI-60 cell line, EC50 values for the 4 post-infection time points 24, 48, 72 and 96 hours. In step one of the approach, one-way ANOVA followed by post hoc Dunnett contrast testing (Dunnett 1955) of the contrast 'MOI 0 versus MOI 50' was carried out to assess whether H-1PV amount has a consistent effect on normalized cell index. No consistent effect was concluded if (a) ANOVA failed to demonstrate a global effect of H-1PV amount on normalized cell index (p>0.05) or (b) a global but inconsistent effect was revealed by ANOVA (p≤0.05) followed by post hoc Dunnett contrast test (p>0.05). No Dunnett contrast testing was needed and was thus not performed for case (a). If no consistent effect was found in step one, no EC50 value was reported for the respective combination of NCI-60 cancer cell line and post-infection time point. Otherwise, in step two of the approach, the EC50 value was computed by fitting the 4-parameter log-logistic model to the concentration-response data (concentration: H-1PV amount, response: normalized cell index) that was obtained for the respective combination of NCI-60 cancer cell line and post-infection time point. As negative values for the normalized cell index are biologically not meaningful, the lower asymptote of the 4-parameter log-logistic model function was restricted to be ≥ 0. No EC50 value was reported in four situations: (1) if the estimated EC50 value exceeded the maximum H-1PV amount tested (e.g., MOI 50), the obtained EC50 estimate was considered unreliable and was thus not reported; (2) if the distance between the lower asymptote c and the upper asymptote d of the fitted concentration-response curve was too small (e.g., if c > 0.7*d), the observed effect of H-1PV amount on normalized cell index was considered to be irrelevant and the EC50 estimate was thus not reported; (3) if an increasing concentration-response curve which is not interpretable from a biological point of view was obtained from the log-logistic model fit; and (4) in situations in which the 4-parameter log-logistic model function failed to fit the concentration-response data. EC50 computation was conducted with the open-source statistical software environment R, version 2.14.2 (http://www.R-project.org) (The statistical analysis was carried out by Sven Stanzel, Department of Statistics, DKFZ, Heidelberg).

### Example 2

### Screening the NCI-60 cancer cell lines for H-1PV cytotoxicity

To identify putative tumors susceptible to H-1PV cytotoxicity the NCI-60 cancer cell line collection, which includes tumor cell lines of different origins (melanoma, lung, colon, central nervous system, ovarian, renal, breast, leukaemia and prostate cancers) was screened. Cells were seeded in a 96-well E-plate and infected with increasing amounts of H-1PV wild-type, ranging from multiplicity of infection [MOI = pfu/cell] of 0.05 to 50 pfu/cell. Cellular proliferation (expressed as normalized cell index, CI) reflecting virus-mediated cytopathic and cytostatic effects, was monitored in real time, every 30 min, using the xCELLigence System (Roche Diagnostics Deutschland GmbH, Mannheim, Germany) for a total of 5-7 days.

The CI values obtained with the xCELLigence analysis were used to determine the EC50 values (viral MOI which kills the 50% of the cell population) at 4 time points after infection (24, 48, 72 and 96 hours). Results from this analysis are reported in Table 1.

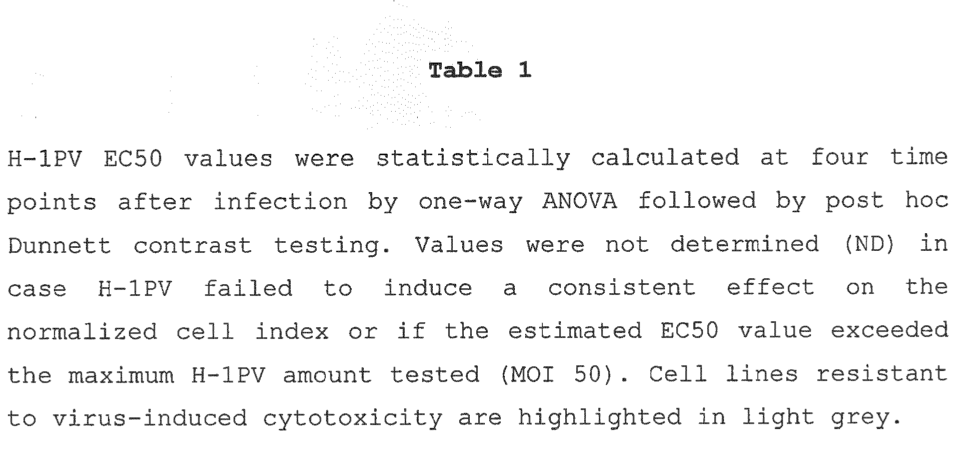

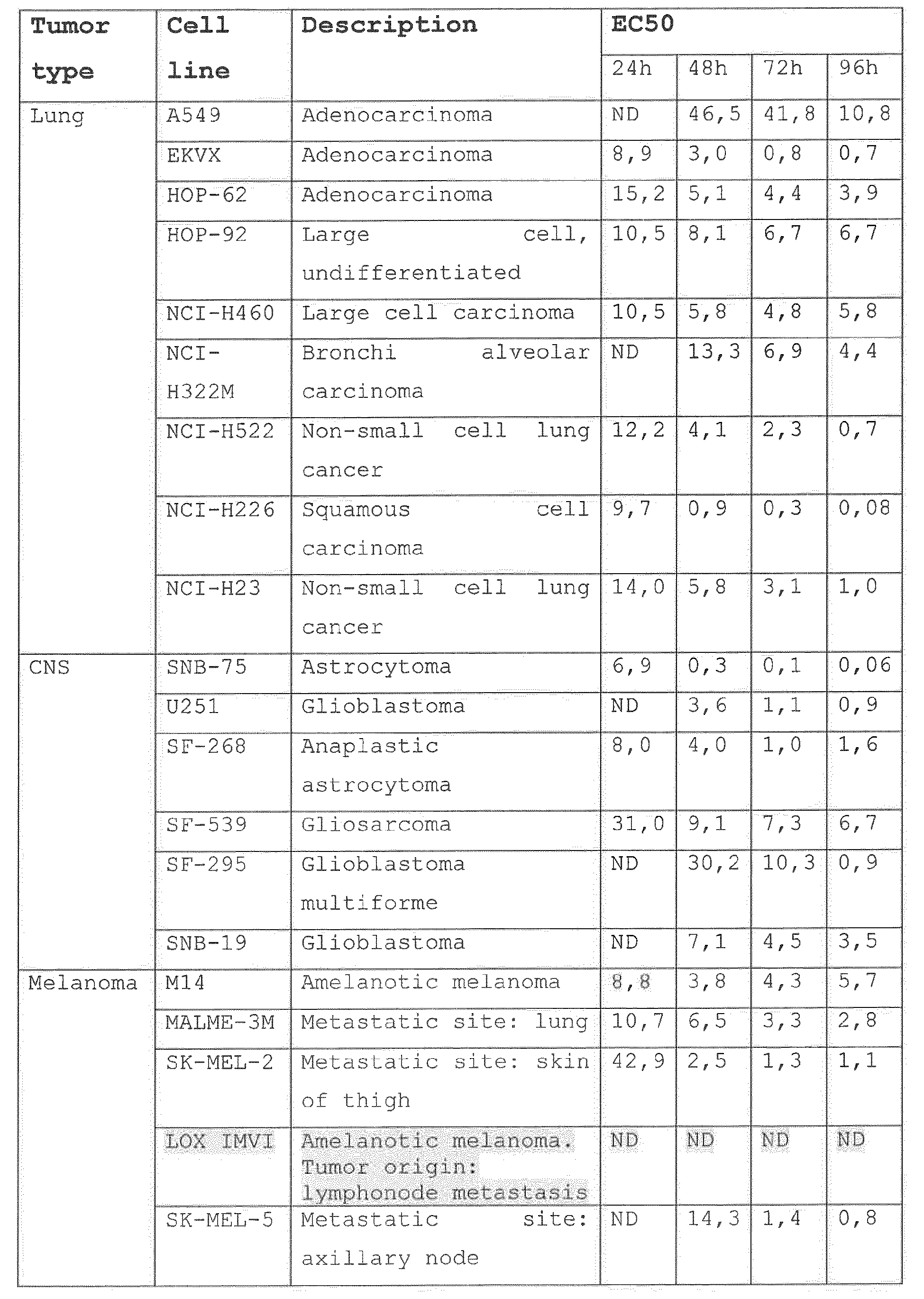

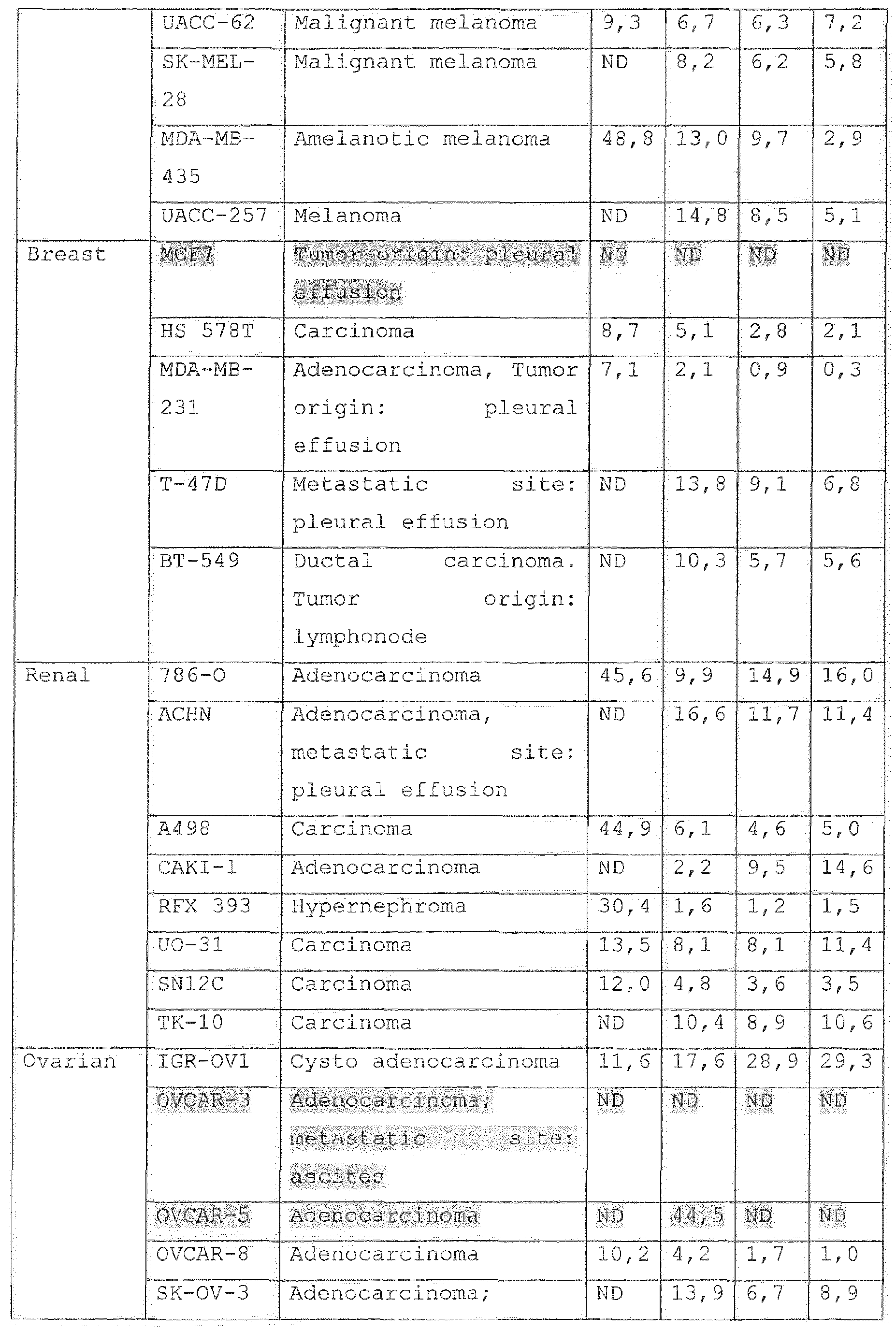

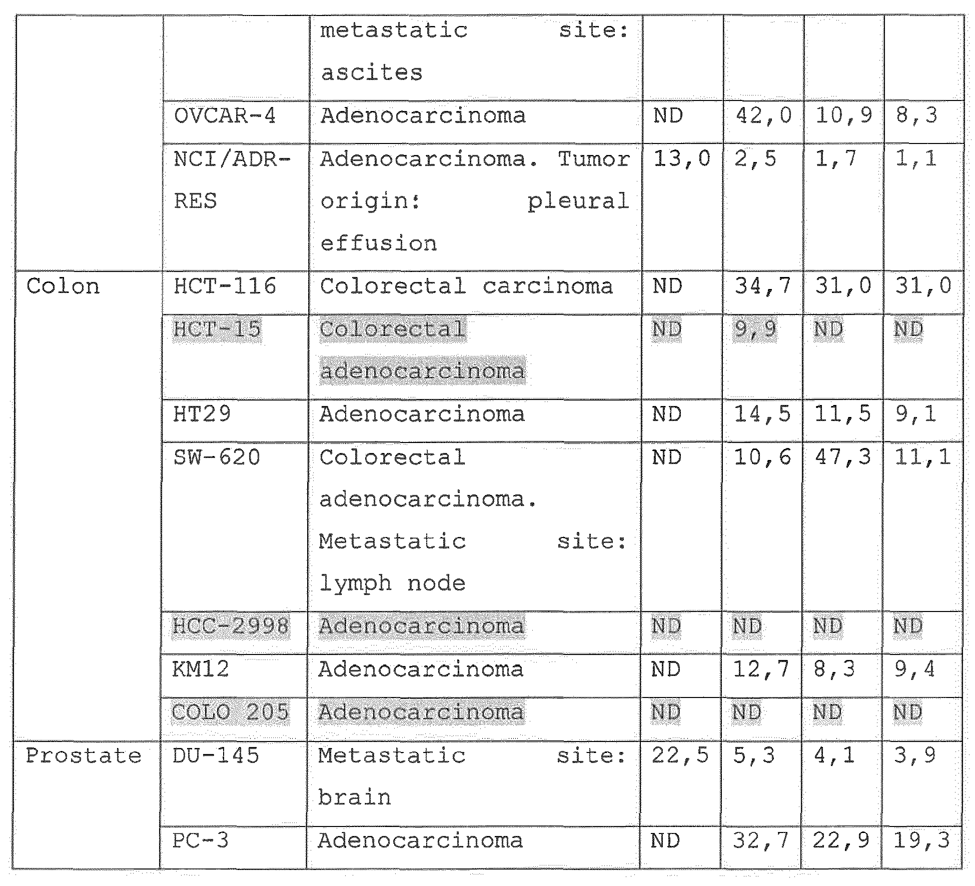

Figure 1 shows results obtained in two cell lines per tumor type (the results of the whole panel are reported in Figures 3 to 10). 37 cell lines of various origins were found to be highly susceptible to H-1PV infectivity (cytostatic and cytotoxic effects were observed at MOIs ≤10), 9 cell lines were low sensitive (killed only when the virus was used at MOI 50), while 7 cell lines were completely refractory to H-1PV infection (including 3 colon carcinomas cell lines). Interestingly, 8 (EKVX, HOP-62, HOP-92, NCI-H226, NCI-H23, NCI-H460, NCI-H322M, NCI-H522) out of 9 cell lines derived from patients with lung carcinomas (adenocarcinomas, large cell carcinomas, squamous and non-small cell lung cancers) were particularly sensitive to H-1PV infection and were efficiently killed by the virus at low MOIs (≤10) already after 48-72 hours from infection, while lung carcinoma A549 cells were killed at a viral MOI of 50 (Figure 3). In general among all the cell lines analyzed, those derived from lung cancer were the most sensitive to parvovirus treatment.

Cell lines derived from patients with melanoma and breast cancer were also highly sensitive to H-1PV cytotoxicity, with only 1 cell line per tumor type being completely refractory (LOX IMVI melanoma cells and the breast cancer cell line MCF7) (Figures 4 and 5). These results confirmed previous published results (Moehler et al, 2005; Muharram et al, 2010), suggesting that breast cancer and melanoma may represent potential targets of H-1PV oncolysis. Virus-induced cytotoxic effects observed in lung, melanoma and breast cancer-derived cell lines correlated with the ability of the virus to transduce these cells with high or moderate efficiency at 24 hours after infection (data not shown). In agreement with previous results (Geletneky et al, 2005; Herrero et al, 2004), cell lines from tumors of CNS origin (glioblastoma, gliosarcoma, astrocytoma) were highly susceptible to H-1PV infection (all the cell lines were killed by the virus at low MOIs after 48-72 hours) (Figure 6). Ovarian cancer cell lines showed medium-low sensitivity to H-1PV oncotoxicity, with 4 cell lines (OVCAR-8, SK-OV-3, OVCAR-4, NCI/ADR-RES) out of 7 being sensitive at MOIs ≤10, 1 cell line (IGR-OV1) sensitive at MOI 50 after 24 hours, while 2 cell lines were resistant (OVCAR-3 and OVCAR-5) (Figure 7). A similar profile was observed in renal cancer-derived cells, of which ACHN, CAKI-1, UO-31 and TK-10 cell lines could be efficiently killed after 24 hours using a MOI of 50 pfu/cell, while other 4 cell lines (786-0, A498, RFX 393, SN12C) were sensitive at MOIs ≤10 (Figure 8). On the contrary, cell lines deriving from colon cancer were particularly resistant to parvovirus cytotoxicity, with 3 cell lines totally refractory (HCT-15, HCC-2998, COLO 205), 2 cell lines (HCT-116 and HT-29) killed by the virus at MOI 50 after 24-48 hours, while in other 2 cell lines (KM12 and SW-620) cytostatic/cytotoxic effects could be observed when the virus was used at MOIs ≤10 (Figure 9). A classification of the prostate cancer as a permissive or resistant tumor entity to H-1PV oncolysis was not possible as the panel only includes 2 cell lines of this origin, with one (DU-145) showing sensitivity at MOI 5, while in the other (PC-3) a MOI of 50 pfu/cell was required to induce cytotoxic effects (Figure 10).

### Example 3

### LDH and MTT assays confirm susceptibility of lung cancer and melanoma derived cell lines to H-1PV infection

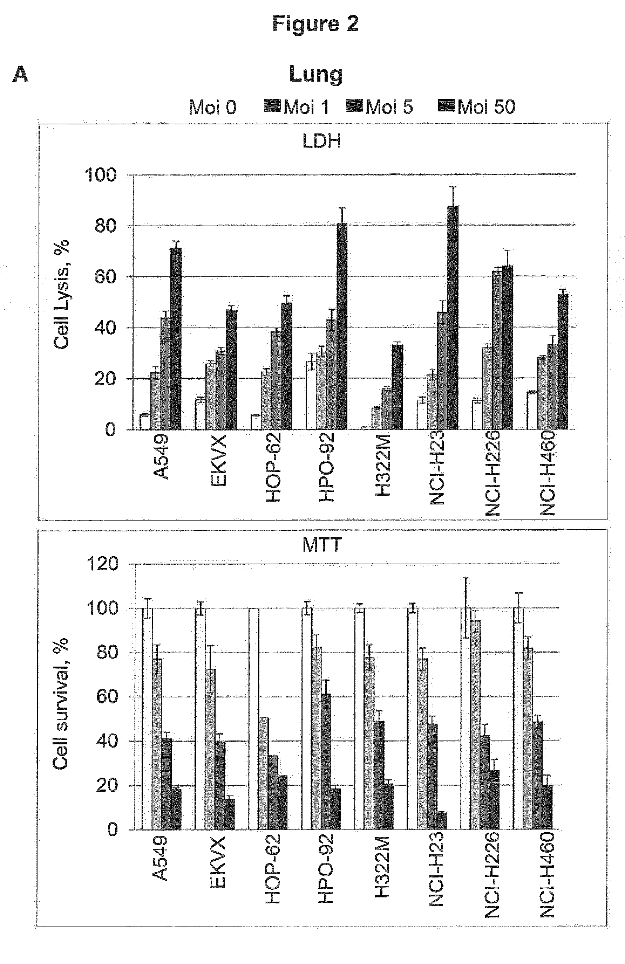

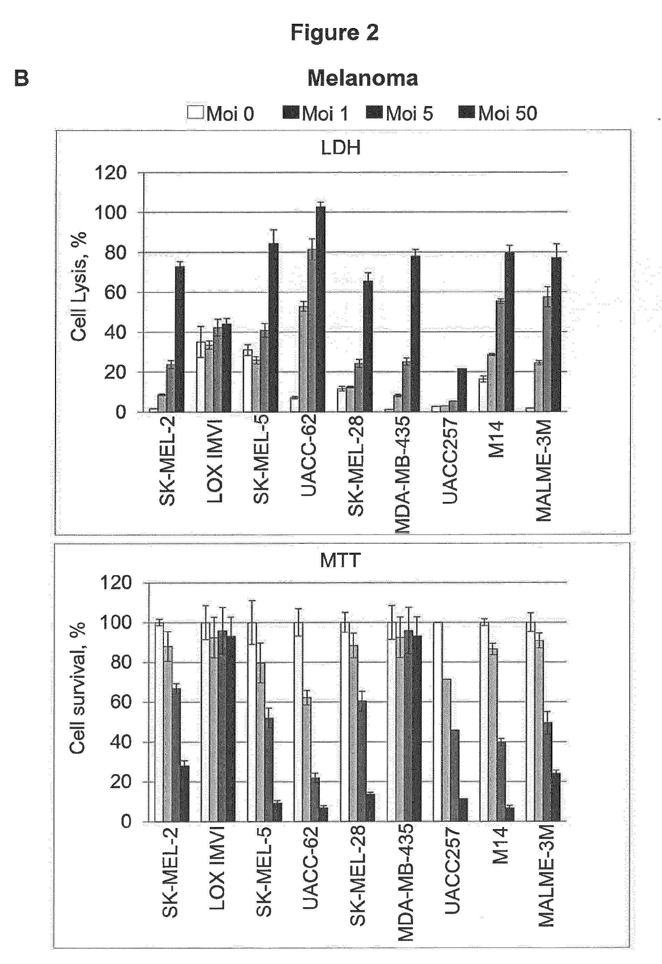

In order to confirm the results obtained with the xCELLigence system, LDH and MTT assays (which measure cell lysis and cell viability, respectively) were performed in lung cancer and melanoma cell lines, selected because of their high susceptibility to H-1PV oncolysis. Cells were plated in 96-well plates and infected with increasing concentrations of H-1PV (MOIs of 0, 1, 5 and 50 pfu/cell) before being processed for LDH and MTT at 48 hours post-infection. As shown in Figure 2, both lung cancer and melanoma-derived cell lines showed high sensitivity to H-1PV cytotoxicity.

In summary, the screening of the NCI-60 panel confirmed the susceptibility of breast, melanoma and CNS-derived cancer cell lines to H-1PV oncotoxicity and showed, for the first time, that lung tumors may represent novel potential targets of H-1PV oncolysis, while colon cancer cell lines are, within this cell lines panel, the most resistant to the virus cytotoxicity.

### List of References

Cornelis JJ, Salome N, Dinsart C, Rommelaere J (2004) Vectors based on autonomous parvoviruses: novel tools to treat cancer? J Gene Med 6 Suppl 1: S 193-202
Cotmore SF, Tattersall P (2007) Parvoviral host range and cell entry mechanisms. Adv Virus Res 70: 183-232
Geletneky K, Herrero YCM, Rommelaere J, Schlehofer JR (2005) Oncolytic potential of rodent parvoviruses for cancer therapy in humans: a brief review. J Vet Med B Infect Dis Vet Public Health 52: 327-330
Herrero YCM, Cornelis JJ, Herold-Mende C, Rommelaere J, Schlehofer JR, Geletneky K (2004) Parvovirus H-1 infection of human glioma cells leads to complete viral replication and efficient cell killing. International journal of cancer Journal international du cancer 109: 76-84
Kinsner-Ovaskainen A, Prieto P, Stanzel S, Kopp-Schneider A (2013) Selection of test methods to be included in a testing strategy to predict acute oral toxicity: an approach based on statistical analysis of data collected in phase 1 of the ACuteTox project. Toxicology in vitro : an international journal published in association with BIBRA 27: 1377-1394
Marchini A, Bonifati S, Scott EM, Angelova AL, Rommelaere J (2015) Oncolytic parvoviruses: from basic virology to clinical applications. Virol J12: 6
Moehler MH, Zeidler M, Wilsberg V, Cornelis JJ, Woelfel T, Rommelaere J, Galle PR, Heike M (2005) Parvovirus H-1-induced tumor cell death enhances human immune response in vitro via increased phagocytosis, maturation, and cross-presentation by dendritic cells. Human gene therapy 16: 996-1005
Muharram G, Le Rhun E, Loison I, Wizla P, Richard A, Martin N, Roussel A, Begue A, Devos P, Baranzelli MC, Bonneterre J, Caillet-Fauquet P, Stehelin D (2010) Parvovirus H-1 induces cytopathic effects in breast carcinoma-derived cultures. Breast cancer research and treatment 121: 23-33
Nuesch JP, Lacroix J, Marchini A, Rommelaere J (2012) Molecular pathways: rodent parvoviruses--mechanisms of oncolysis and prospects for clinical cancer treatment. Clin Cancer Res 18: 3516-3523
Rommelaere J, Geletneky K, Angelova AL, Daeffler L, Dinsart C, Kiprianova I, Schlehofer JR, Raykov Z (2010) Oncolytic parvoviruses as cancer therapeutics. Cytokine Growth Factor Rev 21: 185-195
Russell SJ, Peng KW, Bell JC (2012) Oncolytic virotherapy. Nat Biotechnol 30: 658-670

## Claims

1. Parvovirus H1 (H-1PV) or a related rodent parvovirus selected from the group consisting of LuIII, Mouse minute virus (MMV), Mouse parvovirus (MPV), Rat minute virus (RMV), Rat parvovirus or Rat virus (RV) for use in treating lung cancer.

2. Parvovirus H1 (H-1PV) or a related rodent parvovirus selected from the group consisting of LuIII, Mouse minute virus (MMV), Mouse parvovirus (MPV), Rat minute virus (RMV), Rat parvovirus or Rat virus (RV) for the use according to claim 1 **characterized in that** the use is for treating non-small cell lung cancer.

3. Parvovirus H1 (H-1PV) or a related rodent parvovirus selected from the group consisting of LuIII, Mouse minute virus (MMV), Mouse parvovirus (MPV), Rat minute virus (RMV), Rat parvovirus or Rat virus (RV) for the use according to claim 1 or 2 **characterized in that** the parvovirus is combined with a chemotherapeutic agent.
